# EUROPEAN PATENT SPECIFICATION

(11) **EP 3 003 491 B1**
(45) Date of publication and mention of the grant of the patent: **02.01.2019**
(21) Application number: 14729266.8
(22) Date of filing: 02.06.2014
(51) Int. Cl.: A61Q 1/02, A61K 8/87, A61K 8/06, A61K 8/29, A61K 8/44, A61K 8/02, A61K 8/19

(54) **COSMETIC COMPOSITIONS COMPRISING AN ASSOCIATIVE POLYURETHANE AND A HYDROPHOBIC COATED PIGMENT**
KOSMETISCHE ZUSAMMENSETZUNGEN MIT ASSOZIATIVEM POLYURETHAN UND EINEM HYDROPHOB BESCHICHTETEN PIGMENT
COMPOSITIONS COSMÉTIQUES COMPRENANT UN POLYURÉTHANE ASSOCIATIF ET UN PIGMENT À REVÊTEMENT HYDROPHOBE

(30) Priority: 03.06.2013 FR 1355067; 03.06.2013 FR 1355069
(43) Date of publication of application: 13.04.2016
(73) Proprietor: L'OREAL, 75008 Paris (FR)
(72) Inventor: BRUN, Gaëlle, 92330 Sceaux (FR); COLLETTE, Annick, F-94100 St Maur Des Fosses (FR); VALVERDE, Elodie, 75012 Paris (FR); LEZORAY, Anne-Marie, 91580 Villeconin (FR)
(74) Representative: Lavoix
(86) International application number: PCT/EP2014/061370
(87) International publication number: WO 2014/195265

(56) References cited:
- EP-A1- 0 970 692
- EP-A1- 1 380 287
- EP-A1- 1 844 756
- FR-A1- 2 861 986

## Description

The present invention relates to cosmetic compositions comprising at least one associative polyurethane and at least one hydrophobic coated pigment, in the form of oil-in-water emulsions. These compositions are particularly intended for skin care and/or makeup.

Most foundations are water-in-oil emulsions resulting in a lack of freshness on application.

Foundations which are in oil-in-water emulsion form have satisfactory freshness but poor application properties with a relatively short application time, low degree of slip and a friction sensation perceived at the end of application. According to one particular embodiment, the surface of the composition may be self-smoothing, meaning that it smoothes again a few moments after the product has been extracted.

Adding an associative polyurethane to a foundation in direct emulsion helps enhance application considerably. However, such a foundation does not always adhere well to the skin, it remains relatively difficult to spread on the face and the application time is relatively short.

Therefore, there is currently a need for a foundation type formulation in the form of an oil-in-water emulsion having satisfactory application properties on the skin.

The aim of the present invention is thus that of providing oil-in-water emulsions with satisfactory cosmetic application properties. The aim of the present invention is also that of providing oil-in-water emulsions comprising an associative polyurethane suitable for concurrently having satisfactory application and adhesion properties on the skin and a satisfactory macroscopic appearance of the formula.

The aim of the present invention is also that of providing oil-in-water emulsions suitable for enhancing the slip of compositions comprising an associative polyurethane, while enhancing the makeup properties of said compositions.

In this way, the present invention relates to a cosmetic composition comprising a physiologically acceptable medium, in the form of an oil-in-water emulsion, containing:
- at least one associative polyurethane complying with the following formula (I): 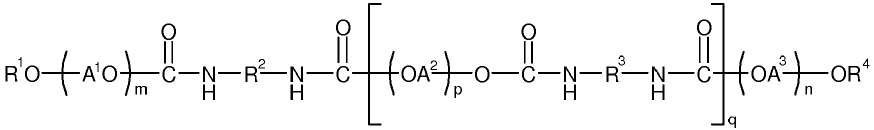 in which:
   * R¹ and R⁴ represent, independently of each other, a linear hydrocarbon radical comprising 1 to 30 carbon atoms,
   * A¹, A² and A³ represent, independently of each other, a linear or branched alkylene radical, having 2 to 4 carbon atoms;
   * m and n represent, independently of each other, an integer between 35 and 500,
   * p represents an integer between 5 and 500,
   * q represents an integer between 1 and 8,
   * R² and R³ represent, independently of each other, a linear or branched bivalent hydrocarbon radical, comprising 1 to 30 carbon atoms,
- and pigment particles coated with at least one lipophilic compound,
the average size of said particles being greater than 100 nm.

By means of the associative polyurethane, a composition with a thick texture, which spreads well, is rheofluidizable (wherein the viscosity decreases with the shear rate) and has remarkable viscoelasticity is obtained.

It has been demonstrated that associating the particular associative polyurethane with hydrophobically coated pigment particles makes it possible to enhance foundation application considerably, reduce the adhesion problem and enhance the slip of the formula and the application time thereof.

Furthermore, the presence of hydrophobic coated pigment makes it possible to obtain a makeup result with more cover and thus closer to the makeup result obtained with a conventional water-in-oil foundation. As the result offers more cover, facial color imperfections are toned down more, giving rise to a superior makeup result.

### Associative polyurethane

According to the present invention, the term "associative polyurethane" denotes an amphiphilic polymer capable, in an aqueous medium, of reversibly associating with itself or with other molecules. It generally comprises in the chemical structure thereof at least one hydrophilic region or group and at least one hydrophobic region or group.

Associative polyurethanes are non-ionic sequenced copolymers comprising in the chain, both hydrophilic sequences generally polyoxyethylenated in nature and hydrophobic sequences which may be aliphatic chains alone and/or cycloaliphatic and/or aromatic chains.

In particular, these polymers comprise at least two hydrocarbon lipophilic chains, having 6 to 30 carbon atoms, separated by a hydrophilic sequence, the hydrocarbon chains may be pendant chains or hydrophilic sequence end chains. In particular, one or a plurality of pendant chains may be envisaged. Moreover, the polymer may comprise a hydrocarbon chain at one end or at both ends of a hydrophilic sequence.

The polymers may be sequenced in triblock or multiblock form. The hydrophobic sequences may thus be at each end of the chain (for example: triblock copolymer having hydrophilic central sequence) or distributed both at the end and in the chain (multisequenced copolymer for example). The polymers may also be grafted or star polymers.

Preferentially, the associative polyurethanes according to the invention are triblock copolymers wherein the hydrophilic sequence is a polyoxyethylenated chain comprising 50 to 1000, particularly 100 to 300, oxyethylenated groups; and comprising at least two hydrocarbon lipophilic chains having 6 to 30 carbon atoms, separated by said hydrophilic sequence, said hydrocarbon lipophilic chains may be pendant chains or hydrophilic sequence end chains.

Preferably, the associative polyurethanes according to the invention have a mean molecular weight by mass (Mw) less than or equal to 500,000 g/mol, more preferably less than or equal to 100,000 g/mol.

The cosmetic compositions according to the invention comprise an associative polyurethane having formula (I) as defined above. They may also comprise a plurality of associative polyurethanes as defined above.

According to one embodiment, the cosmetic compositions according to the invention comprise 0.01% to 10% by weight of associative polyurethane active substance having formula (I) in relation to the total weight of said composition. Preferably, the associative polyurethane content ranges from 0.1% to 5% by weight, and preferentially from 0.1% to 4% by weight of active substance, in relation to the total weight of said composition.

In formula (I) as defined above, R¹ and R⁴ represent, independently of each other, a linear hydrocarbon radical comprising 1 to 30 carbon atoms, i.e. a linear radical consisting of carbon atoms and hydrogen atoms.

The term "hydrocarbon radical" denotes a radical comprising only carbon and hydrogen atoms, and optionally comprising one or a plurality of unsaturations.

In formula (I), the terminal groups R¹ and R⁴ are linear chains, which may be chosen for example from the alkyl, alkenyl, or alkynyl groups.

According to one embodiment, in formula (I) as defined above, R¹ and R⁴ are hydrocarbon linear radicals comprising 8 to 30 carbon atoms.

According to one preferred embodiment, R¹ and R⁴ are linear alkyl groups comprising 1 to 30 carbon atoms. Preferentially, in formula (I), R¹ and R⁴ represent, independently of each other, a linear alkyl group comprising 8 to 30 carbon atoms.

According to one embodiment, in formula (I), R¹ and R⁴ represent a linear alkyl group comprising 18 carbon atoms.

In formula (I), each of A¹, A² and A³ is an alkylene divalent radical which may be linear or branched, comprising 2, 3 or 4 carbon atoms. These alkylene radicals may be ethylene, propylene or butylene groups such as isobutylene.

According to one embodiment, in formula (I), each of radicals A¹, A² and A³ represents an ethylene radical.

According to one embodiment, in formula (I), m and n represent, independently of each other, an integer between 50 and 200. According to one particular embodiment, m=n=100.

According to one embodiment, in formula (I), p represents an integer between 50 and 200. According to one particular embodiment, in formula (I), p=136.

According to one embodiment, in formula (I), q represents an integer between 1 and 3. Preferably, in formula (I), q=1.

In formula (I), R² and R³ represent, independently of each other, a linear or branched bivalent hydrocarbon radical, corresponding to a diisocyanate residue having the respective formulas OCN-R²-NCO and OCN-R³-NCO.

These radicals comprise 1 to 30 carbon atoms, preferably 6 to 20 carbon atoms.

In particular, the radicals R² and R³ may be aliphatic, alicyclic or aromatic radicals.

According to one preferred embodiment, the radicals R² and R³ are the residues of the diisocyanates R²-(NCO)₂ and R³-(NCO)₂, these diisocyanates being chosen from aliphatic diisocyanates, aromatic diisocyanates, alicyclic diisocyanates, biphenyl diisocyanates, and phenylmethane diisocyanates.

Of the preferred diisocyanates, particular mention may be made of the following compounds: 2,4-tolylene diisocyanate, 2,6-tolylene diisocyanate, hexamethylene diisocyanate, 4,4'-diphenylmethane diisocyanate, 4,4'-diphenyl diisocyanate, 1,4-phenylene diisocyanate, and more particularly the following diisocyanates: trimethyl hexamethylene diisocyanate, isophorone diisocyanate, methylene-bis-(4-cyclohexyl)-diisocyanate, and more preferentially 1,6-hexamethylene diisocyanate (HDI).

Preferably, in formula (I), R² and R³ are hexamethylene radicals. According to this embodiment, the corresponding diisocyanate is thus hexamethylene diisocyanate (HDI).

Within the scope of the present invention, the preferred associative polyurethane is Rheolate FX1100® supplied by ELEMENTIS. This polyurethane is a polycondensate of polyethylene glycol having 136 moles of ethylene oxide, polyoxyethylenated stearyl alcohol having 100 moles of ethylene oxide and hexamethylene diisocyanate (HDI)(INCI name: PEG- 136/Steareth-100I/HDI Copolymer).

As the associative polyurethane according to the invention, mention may also be made of an associative polyurethane suitable for being obtained by means of polycondensation of at least three compounds comprising (i) at least one polyethylene glycol comprising 150 to 180 moles of ethylene oxide, (ii) stearyl alcohol or decyl alcohol and (iii) at least one diisocyanate.

Such polymers are particularly supplied by ROHM & HAAS under the names Aculyn 46 ® and Aculyn 44 ®.

ACULYN 46® is a polycondensate of polyethylene glycol having 150 or 180 moles of ethylene oxide, stearyl alcohol and methylene bis(4-cyclohexyl-isocyanate) (SMDI) at 15% by weight in a matrix of maltodextrin (4%) and water (81%)(INCI name: PEG-150/STEARYL ALCOHOL/SMDI COPOLYMER).

ACULYN 44® is a polycondensate of polyethylene glycol having 150 or 180 moles of ethylene oxide, decyl alcohol and methylene bis(4-cyclohexyl-isocyanate) (SMDI), at 35% by weight in a matrix of propylene glycol (39%) and water (26%)(INCI name: PEG-150/DECYL ALCOHOL/SMDI COPOLYMER).

### Coated pigment and/or nacre particles

The cosmetic compositions according to the invention comprise pigment particles coated with a lipophilic compound, these particles may be
identical or different. The compositions according to the invention may thus comprise mixtures of pigment particles of different types.

The particle size of the pigment is strictly greater than 100 nm.

According to the invention, the term "size" of a particle denotes the D50 thereof. The D50, or median size by volume, corresponds to the particle size defined such that 50% by volume of the particles have a size less than D50.

The median size by volume may be assessed by means of light diffraction using a Malvern MasterSizer laser granulometer, said particles under evaluation being dispersed in a liquid medium such as for example octyldodecyl neopentanoate. According to one embodiment, the size of the pigment particles
according to the invention ranges from 100 nm to 25µm, preferably from 200 nm to 10µm.

A composition according to the invention thus comprises
at least
one pigment coated with at least one lipophilic compound.

The pigments are coated hydrophobically so as to be situated in
the oil phase of the emulsion, i.e. in the internal phase.

The term "pigments" should be understood to mean white or colored, mineral or organic particles, which are insoluble in an aqueous solution and are intended for coloring and/or opacifying the resulting composition.

The coated pigment particles are iron oxide and/or titanium oxide particles coated with a lipophilic compound chosen from the group consisting of disodium stearoyl glutamate, isopropyl trisostearyl titanate, dimethicone, triethoxy caprylylsilane, hydrogenated lecithin and mixtures thereof.

Preferably, the coated pigments used as iron oxides and titanium oxides coated with Disodium Stearoyl Glutamate (NAI), isopropyl trisostearyl titanate (ITT), Dimethicone (SA), Triethoxy caprylylsilane (AS) or Hydrogenated Lecithin (HLC).

The composition according to the present invention may further comprise one or a plurality of non-surface-treated pigments or nacres (additional dyes).

### Hydrophilic gelling agent

The cosmetic compositions according to the invention may also comprise at least one hydrophilic gelling agent, chosen from the group consisting of cross-linked and/or neutralized polyacrylamides and polymers and copolymers of 2-acrylamido 2-methylpropane sulfonic acid, polysaccharides and mixtures thereof.

Adding a hydrophilic gelling agent to the composition may help enhance the stability of the composition particularly at high temperatures.

According to one embodiment, the mass ratio between the hydrophilic gelling agent and the associative polyurethane is less than 1, preferably between 1:50 and 1, and more preferentially between 1:20 and 1:2.

When the hydrophilic gelling agent content is too high, the cosmetic properties of the composition according to the invention are diminished.

The cosmetic compositions according to the invention preferably comprise a hydrophilic gelling agent as defined above. They may also comprise a mixture of a plurality of hydrophilic gelling agents as defined above.

The cosmetic compositions according to the invention may comprise 0.01% to 10%, preferably 0.1% to 5%, and preferentially 0.1% to 3% by weight of hydrophilic gelling agent active substance in relation to the total weight of said composition.

Within the scope of the present invention, the gelling agent is chosen as defined above so as not to degrade the satisfactory application properties provided by the associative polyurethane. In this way, the preferred gelling agent should have a satisfactory rheofluidizing potential to have the least possible effect on the viscosity of the formula under shearing during applications.

The gelling agent should further be compatible with the associative polyurethane. The main purpose of the gelling agent is thus that of increasing the viscosity of the formula at low shear levels particularly for high temperatures.

### A- Cross-linked and/or neutralized polyacrylamides and polymers and copolymers of 2-acrylamido 2-methylpropane sulfonic acid

The hydrophilic gelling agents may be cross-linked or non-cross-linked homopolymers or copolymers comprising at least the acrylamido 2-methylpropane sulfonic acid monomer (AMPS®), in a partially or completely neutralized form by a mineral base other than ammonia such as soda or potash.

They are preferably completely or practically completely neutralized, i.e. at least 90% neutralized.

These AMPS® polymers according to the invention may be cross-linked or non-cross-linked.

When the polymers are cross-linked, the cross-linking agent may be chosen from the olefinic polyunsaturation compounds routinely used for cross-linking polymers obtained by radical polymerization.

As cross-linking agents, mention may be made, for example, of divinylbenzene, diallyl ether, dipropyleneglycol-diallylether, polyglycol-diallylethers, triethyleneglycol-divinylether, hydroquinone-diallyl-ether, ethyleneglycol or tetraethyleneglycol di(meth)acrylate, trimethylol propane triacrylate, methylene-bis-acrylamide, methylene-bis-methacrylamide, triallylamine, triallylcyanurate, diallylmaleate, tetraallylethylenediamine, tetra-allyloxy-ethane, trimethylolpropane-diallylether, allyl (meth)acrylate, sugar alcohol series allyl ethers, or other polyfunctional alcohol allyl- or vinyl- ethers, and phosphoric and/or vinylphosphoric acid derivative allyl esters, or mixtures of these compounds.

According to one preferred embodiment of the invention, the cross-linking agent is chosen from methylene-bis-acrylamide, allyl methacrylate or trimethylol propane triacrylate (TMPTA). The degree of cross-linking generally ranges from 0.01% to 10% in moles and more particularly from 0.2% to 2% in moles in relation to the polymer.

The AMPS® polymers suitable for the invention are water-soluble or water-dispersible. They are in this case:
- either "homopolymers" only comprising AMPS monomers and, if they are cross-linked, one or a plurality of cross-linking agents such as those defined above;
- or copolymers obtained from AMPS® and one or a plurality of hydrophilic or hydrophobic monomers with ethylene unsaturation and, if they are cross-linked, one or a plurality of cross-linking agents such as those defined above. When said copolymers comprise hydrophobic monomers with ethylene unsaturation, the latter do not comprise fat chains and are preferably present in small quantities.

The term "fat chain" denotes, according to the present invention, any hydrocarbon chain comprising at least 7 carbon atoms.

The term "water-soluble or water-dispersible" denotes polymers which, introduced into an aqueous phase at 25°C, at a mass concentration equal to 1%, make it possible to obtain a macroscopically homogeneous and transparent solution, i.e. having a maximum light transmittance value, at a wavelength equal to 500 nm, through a 1 cm thick sample, of at least 60%, preferably at least 70%.

The "homopolymers" according to the invention are preferably cross-linked and neutralized, and they can be obtained according to the preparation method comprising the following steps:
(a) dispersing or dissolving the monomer such as AMPS in free form in a tertio-butanol or water and tertio-butanol solution;
(b) neutralizing the monomer solution or dispersion obtained in (a) with one or a plurality of mineral or organic bases, preferably ammonia NH₃, in a quantity suitable for obtaining a neutralization rate of the sulfonic acid functions of the polymer ranging from 90 to 100%;
(c) adding to the solution or dispersion obtained in (b), the cross-linking monomer(s);
(d) performing conventional radical polymerization in the presence of free radical initiators at a temperature ranging from 10°C to 150°C; the polymer precipitating in the solution or dispersion based on tertio-butanol.

The water-soluble or water-dispersible AMPS® copolymers according to the invention contain water-soluble monomers with ethylene unsaturation, hydrophobic monomers or mixtures thereof.

The water-soluble co-monomers may be ionic or non-ionic.

Of the ionic water-soluble co-monomers, mention may be made, for example, of the following compounds and salts thereof:
- (meth)acrylic acid,
- styrene sulfonic acid,
- vinylsulfonic acid and (meth)allylsulfonic acid,
- vinyl phophonic acid,
- maleic acid,
- itaconic acid,
- crotonic acid,
- water-soluble vinyl monomers having the following formula (A): 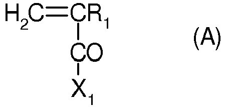 in which:
- R₁ is chosen from H, -CH₃, -C₂H₅ or -C₃H₇;
- X₁ is chosen from alkyl oxides of the type -OR₂ where R₂ is a linear or branched, saturated or unsaturated hydrocarbon radical, having 1 to 6 carbon atoms, substituted by at least one sulfonic (-SO₃-) and/or sulfate (-SO₄-) and/or phosphate group (-PO₄H₂-).

Of the non-ionic water-soluble co-monomers, mention may be made, for example, of:
- (meth)acrylamide,
- N-vinylacetamide and N-methyl N-vinylacetamide,
- N-vinylformamide and N-methyl N-vinylformamide,
- maleic anhydride,
- vinylamine,
- N-vinyl lactams comprising a cyclic alkyl group having 4 to 9 carbon atoms, such as N-vinylpyrrolidone, N-butyrolactam and N-vinylcaprolactam,
- vinyl alcohol having the formula CH₂=CHOH,
- water-soluble vinyl monomers having the following formula (B): 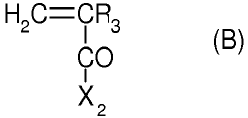 in which:
- R₃ is chosen from H, -CH₃, -C₂H₅ or -C₃H₇;
- X₂ is chosen from alkyl oxides of the type -OR₄ where R₄ is a linear or branched, saturated or unsaturated hydrocarbon radical, having 1 to 6 carbons, optionally substituted by a halogen atom (iodine, bromine, chlorine, fluorine), a hydroxy (-OH) or ether group.

Mention may be made, for example, of glycidyl (meth)acrylate, hydroxyethyl methacrylate, and ethylene glycol, diethyleneglycol or polyalkyleneglycol (meth)acrylates.

Of the hydrophobic comonomers with no fat chain, mention may be made, for example, of:
- styrene and derivatives thereof such as 4-butylstyrene, alpha methylstyrene and vinyltoluene;
- vinyl acetate having the formula CH₂=CH-OCOCH₃;
- vinylethers having the formula CH₂=CHOR wherein R is a linear or branched, saturated or unsaturated hydrocarbon radical, having 1 to 6 carbons;
- acrylonitrile;
- caprolactone;
- vinyl chloride and vinylidene chloride;
- silicone derivatives, resulting after polymerization in silicone polymers, such as methacryloxypropyltris(trimethylsiloxy)silane and silicone methacrylamides;
- hydrophobic vinyl monomers having the following formula (C): 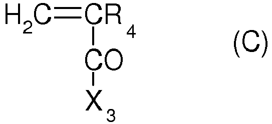 in which:
- R₄ is chosen from H, -CH₃, -C₂H₅ or -C₃H₇;
- X₃ is chosen from alkyl oxides of the type -OR₅ where R₅ is a linear or branched, saturated or unsaturated hydrocarbon radical, having 1 to 6 carbon atoms.

Mention may be made, for example, of methyl methacrylate, ethyl methacrylate, n-butyl (meth)acrylate, tertio-butyl (meth)acrylate, cyclohexyl acrylate and isobornyl acrylate and ethyl 2-hexyl acrylate.

The water-soluble or water-dispersible AMPS@ polymers according to the invention preferably have a molar mass ranging from 50,000 g/mole to 10,000,000 g/mole, preferably from 80,000 g/mole to 8,000,000 g/mole, and more preferably from 100,000 g/mole to 7,000,000 g/mole.

As water-soluble or water-dispersible AMPS homopolymers suitable for the invention, mention may be made of optionally cross-linked sodium acrylamido-2-methylpropane sulfonate polymers such as that used in the commercial product SIMULGEL 800 (CTFA name: Sodium Polyacryloyldimethyl Taurate), cross-linked ammonium acrylamido-2-methylpropane sulfonate polymers (INCI name: ammonium polydimethyltauramide) such as those described in the patent EP0815928B1 and such as the product sold under the trade name HOSTACERIN AMPS@ by Clariant.

As water-soluble or water-dispersible copolymers according to the invention, mention may be made for example of:
- cross-linked sodium acrylamide/acrylamido-2-methylpropane sulfonate copolymers such as those used in the commercial product SEPIGEL 305 (CTFA name: polyacrylamide/C₁₃-C₁₄ isoparaffin / laureth-7) or that used in the commercial product sold under the name SIMULGEL 600 (CTFA name: acrylamide / sodium acryloyldimethyltaurate / isohexadecane / polysorbate-80) by SEPPIC;
- copolymers of AMPS@ and vinylpyrrolidone or vinylformamide such as that used in the commercial product sold under the name ARISTOFLEX AVC® by CLARIANT (CTFA name: ammonium acryloyldimethyltaurate / VP copolymer) but neutralized with soda or potash;
- copolymers of AMPS@ and sodium acrylate, such as for example the AMPS / sodium acrylate copolymer such as that used in the commercial product sold under the name SIMULGEL EG® by SEPPIC or under the trade name SEPINOV EM as (CTFA name: hydroxyethyl acrylate / sodium acryloyldimethyl taurate copolymer);
- copolymers of AMPS® and hydroxyethyl acrylate, such as for example AMPS® / hydroxyethyl acrylate copolymer such as that used in the commercial product sold under the name SIMULGEL NS® by SEPPIC (CTFA name: hydroxyethyl acrylate / sodium acryloyldimethyltaurate copolymer (and) squalane (and) polysorbate 60 or as the product marketed under the name sodium acrylamido-2-methylpropane sulfonate/hydroxyethylacrylate such as the commercial product SEPINOV EMT 10 (INCI name: hydroxyethyl acrylate/sodium acryloyldimethyl taurate copolymer).

According to one particular embodiment, mention may be made of:
- cross-linked sodium acrylamide/acrylamido-2-methylpropane sulfonate copolymers such as those used in the commercial product SEPIGEL 305 (CTFA name: polyacrylamide/C₁₃-C₁₄ isoparaffin/ laureth-7) or that used in the commercial product sold under the name SIMULGEL 600 (CTFA name: acrylamide / sodium acryloyldimethyltaurate / isohexadecane/polysorbate-80) by SEPPIC;
- cross-linked ammonium acrylamido-2-methylpropane sulfonate polymers (INCI name: ammonium polydimethyltauramide) such as those described in the patent EP0815928B1 and such as the product sold under the trade name HOSTACERIN AMPS® by Clariant or optionally cross-linked sodium acrylamido-2-methylpropane such as that used in the commercial product SIMULGEL 800 (CTFA name: Sodium Polyacryloyldimethyl Taurate),
- - copolymers of AMPS® and hydroxyethyl acrylate, such as for example AMPS®/hydroxyethyl acrylate copolymer such as that used in the commercial product sold under the name SIMULGEL NS® by SEPPIC (CTFA name: hydroxyethyl acrylate / sodium acryloyldimethyltaurate copolymer (and) squalane (and) polysorbate 60 or as the product marketed under the name sodium acrylamido-2-methylpropane sulfonate/hydroxyethylacrylate such as the commercial product SEPINOV EMT 10 (INCI name: hydroxyethyl acrylate/sodium acryloyldimethyl taurate copolymer).

More preferably, the hydrophilic gelling agent is chosen from:
- cross-linked ammonium acrylamido-2-methylpropane sulfonate polymers (INCI name: ammonium polydimethyltauramide) such as those described in the patent EP0815928B1 and such as the product sold under the trade name HOSTACERIN AMPS® by Clariant,
- cross-linked sodium acrylamide/acrylamido-2-methylpropane sulfonate copolymers such as those used in the commercial product SIMULGEL 600 (CTFA name: acrylamide/sodium acryloyldimethyltaurate/ isohexadecane/polysorbate-80) by SEPPIC;
- and mixtures thereof.

### B- Polysaccharides

As a general rule, the polysaccharides according to the invention may be chosen from polysaccharides produced by microorganisms; polysaccharides isolated from algae and polysaccharides from upper plants, such as homogeneous polysaccharides, particularly celluloses and derivatives thereof and fructosans, heterogeneous polysaccharides such as gum arabic, galactomannans, glucomannans, pectins, and derivatives thereof.

In particular, the polysaccharides may be chosen from fructans, gellans, glucans, amylose, amylopectin, glycogen, pullulan, dextrans, celluloses and derivatives thereof, particularly methylcelluloses, hydroxyalkylcelluloses, ethylhydroxyethylcelluloses, and carboxymethylcelluloses, mannans, xylans, lignins, arabans, galactans, galacturonans, compounds based on alginate, chitin, chitosans, glucoronoxylans, arabinoxylans, xyloglucans, glucomannans, pectic acids and pectins, arabinogalactans, carrageenans, agars, glycosaminoglucans, gum arabic, Tragacanth gums, Ghatti gums, Karaya gums, carob gums, galactomannans such as guar gums and non-ionic derivatives, particularly hydroxypropyl guar, and ionic derivatives thereof, biopolysaccharide gums of microbial origin, particularly scleroglucan or xanthan gums, mucopolysaccharides, and particularly chondroitin sulfates and mixtures thereof.

Advantageously, a composition according to the invention comprises as a hydrophilic gelling agent at least one polysaccharide chosen from carrageenans, particularly kappa-carrageenan, gellan gum, agar-agar, xanthan gum, alginate-based compounds, particularly sodium alginate, sceroglucan gum, guar gum, inulin, pullulan, and mixtures thereof.

These polysaccharides may be chemically modified, particularly by urea, urethane groups, or by means of a hydrolysis, oxidation, esterification, etherification, sulfation, phosphation, amination, amidation, alkylation reaction, or by a plurality of these modifications.

The derivatives obtained may be anionic, cationic, amphoteric or non-ionic.

Advantageously, the polysaccharides may be chosen from carrageenans, particularly kappa-carrageenan, gellan gum, agar-agar, xanthan gum, alginate-based compounds, particularly sodium alginate, sceroglucan gum, guar gum, inulin, pullulan, and mixtures thereof.

As a general rule, the compounds of this type, suitable for use in the present invention, are chosen from those which are particularly described in "Encyclopedia of Chemical Technology, Kirk-Othmer, Third Edition, 1982, volume 3, pp. 896-900, and volume 15, pp 439-458", in "Polymers in Nature, by E. A. Mc GREGOR and C. T. GREENWOOD, John Wiley & Sons Editions, Chapter 6, pp 240-328, 1980", in the publication by Robert L. DAVIDSON entitled "Handbook of Water soluble gums and resins" published by Mc Graw Hill Book Company (1980) and in Industrial Gums "Polysaccharides and their Derivatives, Edited by Roy L. WHISTLER, Second Edition, Edition Academic Press Inc.".

According to one particular embodiment, the hydrophilic gelling agent is a polysaccharide chosen from xanthan gum, alginate-based compounds, particularly sodium alginate, sceroglucan gum, guar gum, and mixtures thereof.

According to one particularly preferred embodiment, the hydrophilic gelling agent is xanthan gum.

Xanthan gums have a molecular weight between 1,000,000 g/mol and 50,000,000 g/mol and a viscosity between 0.6 and 1.65 Pa.s for an aqueous composition containing 1% xanthan gum (measured at 25°C with a Brookfield LVT viscometer at 60 rpm).

Xanthan gums are represented for example by the products sold under the names Rhodicare by RHODIA CHIMIE, under the name SATIAXANE™ by Cargill Texturizing Solutions (for the food, cosmetic and pharmaceutical industry), under the name NOVAXAN™ by ADM, and under the names Kelzan® and Keltrol® by CP-Kelco.

To prepare the cosmetic compositions according to the invention, the hydrophilic gelling agent may be introduced before or after the emulsification phase. It is preferably introduced before the emulsification phase at the very start of the process. A gel in water may be pre-prepared or the gelling agent may also be wetted in a portion of the oil of the composition.

### Physiologically acceptable medium

In addition to the compounds indicated above, a cosmetic composition according to the invention includes a physiologically acceptable medium.

The term "physiologically acceptable medium" is intended to denote a medium that is particularly suitable for the application of a composition of the invention to the skin or the lips.

The physiologically acceptable medium is generally suitable for the nature of the support to which the composition should be applied, and also for the way in which the composition is to be packaged.

The cosmetic compositions according to the invention are in the form of an O/W emulsion containing a dispersed fat (or oil) phase and a continuous aqueous phase.

### Aqueous phase

The aqueous phase of the compositions according to the invention comprises water.

According to one embodiment, the aqueous phase of the compositions according to the invention represents at least 50% by weight in relation to the total weight of said composition.

According to one embodiment, the compositions according to the invention comprise at least 40% by weight of water, particularly at least 45% by weight of water in relation to the total weight of said composition.

A water suitable for the invention may be a floral water such as cornflower water and/or a mineral water such as Vittel water, Lucas water or La Roche Posay water and/or a spring water.

The aqueous phase may also comprise water-miscible organic solvents (at ambient temperature - 25°C) such as for example mono-alcohols having 2 to 6 carbon atoms such as ethanol, isopropanol; polyols having particularly 2 to 20 carbon atoms, preferably having 2 to 10 carbon atoms, and preferentially having 2 to 6 carbon atoms, such as glycerol, propylene glycol, butylene glycol, pentylene glycol, hexylene glycol, caprylylglycol, dipropylene glycol, diethylene glycol; glycol ethers (having particularly 3 to 16 carbon atoms) such as mono, di- or tripropylene glycol alkyl(C₁-C₄)ether, mono, di- or triethylene glycol alkyl(C₁-C₄)ethers, and mixtures thereof.

According to one embodiment, the aqueous phase of the compositions according to the invention comprises glycerol, phenoxyethanol, or a mixture thereof.

The aqueous phase may also include any water-soluble or water-dispersible compound compatible with an aqueous phase, such as gelling agents, film-forming polymers, thickeners, surfactants and mixtures thereof.

### Oil phase

The compositions according to the invention also comprise a dispersed oil phase.

According to one embodiment, the oil phase represents 10% to 50%, and preferably 15% to 40%, by weight in relation to the total weight of said composition.

The oil phase (or fat phase) of the compositions according to the invention comprises at least one oil. It may consist of a single oil or a mixture of a plurality of oils. The term "oil" is intended to mean any fatty substance in liquid form at ambient temperature (20-25°C) and at atmospheric pressure. These oils may be of plant, mineral or synthetic origin.

According to one embodiment, the oils are chosen from the group consisting of hydrocarbon oils, silicone oils, fluorinated oils and mixtures thereof.

According to the present invention, the term "hydrocarbon oil" denotes an oil containing mainly hydrogen and carbon atoms.

The term "silicone oil" denotes an oil comprising at least one silicon atom and particularly comprising at least one Si-O group.

The term "fluorinated oil" denotes an oil comprising at least one fluorine atom.

The oils may optionally comprise oxygen, nitrogen, sulfur and/or phosphorus atoms, for example in the form of hydroxyl or acid radicals.

According to one embodiment, the oil phase of the compositions according to the invention comprises at least one volatile oil and/or at least one non-volatile oil.

### Volatile oils

According to one embodiment, the oil phase of the compositions according to the invention comprises at least one volatile oil. The oil phase of the compositions according to the invention may comprise a mixture of a plurality of volatile oils.

The term "volatile oil" denotes any non-aqueous medium capable of evaporating on contact with the skin or the lips, in less than one hour, at ambient temperature and at atmospheric pressure. The volatile oil is a volatile cosmetic oil that is liquid at ambient temperature. More specifically, a volatile oil has an evaporation rate of between 0.01 and 200 mg/cm²/min, inclusive.

To measure this evaporation rate, 15g of oil or an oil mixture to be tested are introduced into a crystallizer with a diameter of 7cm, placed on a scale located in a large chamber of around 0.3 m³ with controlled temperature, at 25°C, and hygrometry, at 50% relative humidity. The liquid is left to evaporate freely, without stirring, by allowing ventilation with a fan (PAPST-MOTOREN, reference 8550 N, rotating at 2700 rpm) arranged vertically above the crystallizer containing said oil or said mixture, with the blades being directed toward the crystallizer and at a distance of 20 cm with respect to the crystallizer base. The mass of oil remaining in the crystallizer is measured at regular intervals. The evaporation rates are expressed in mg of oil evaporated per unit of surface (cm²) and per unit of time (minutes).

The volatile oils may be hydrocarbon, silicone or fluorinated oils.

The volatile oils may be chosen from hydrocarbon oils having 8 to 16 carbon atoms, and particularly branched C₈-C₁₆ alkanes (also known as isoparaffins or isoalkanes), such as isododecane (also known as 2,2,4,4,6-pentamethylheptane), isodecane, isohexadecane, and, for example, the oils sold under the trade names ISOPARS® or PERMETHYLS®.

As hydrocarbon volatile oils, mention may also be made of linear C₉-C₁₇ alkanes, such as dodecane (C₁₂) and tetradecane (C₁₄), marketed respectively under the references PARAFOL® 12-97 and PARAFOL® 14-97 (Sasol) and such as the alkanes obtained according to the method described in the international application WO2007/068371 A1, such as the mixture of undecane (C₁₁) and tridecane (C₁₃).

It is also possible to use, as volatile oils, volatile silicones, such as, for example, volatile linear or cyclic silicone oils, in particular those having a viscosity below or equal to 8 centistokes (cst) (8 x 10⁻⁶ m²/s), and having, in particular, 2 to 10 silicon atoms, and in particular, 2 to 7 silicon atoms, these silicones optionally comprising alkyl or alkoxyl groups having 1 to 10 carbon atoms. Mention may be made, as a volatile silicone oil that can be used in the invention, in particular, of dimethicones with a viscosity of 5 and 6 cSt, octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, dodecamethyl pentasiloxane, and mixtures thereof.

More specifically, as a volatile silicone oil, mention may be made of linear or cyclic silicone oils having 2 to 7 silicon atoms, these silicones optionally comprising alkyl or alkoxyl groups having 1 to 10 carbon atoms.

Mention may be made, as a volatile fluorinated oil, for example, of nonafluoromethoxybutane or perfluoromethylcyclopentane, and mixtures thereof.

### Non-volatile oils

According to one embodiment, the oil phase of the compositions according to the invention comprises at least one non-volatile oil. The oil phase of the compositions according to the invention may comprise a mixture of a plurality of non-volatile oils.

The term "non-volatile oil" denotes an oil remaining on the skin or keratin fiber at ambient temperature and atmospheric pressure. More specifically, a non-volatile oil has an evaporation rate strictly below 0.01 mg/cm²/min.

The non-volatile oils may, in particular, be chosen from non-volatile hydrocarbon, fluorinated and/or silicone oils.

As a non-volatile hydrocarbon oil, mention may be made of:
- hydrocarbon oils of plant origin, such as phytostearyl esters, for instance phytostearyl oleate, phytostearyl isostearate and lauroyl/octyldodecyl/phytostearyl glutamate (AJINOMOTO, ELDEW PS203), triglycerides constituted of glycerol and fatty acid esters, in particular in which the fatty acids may have chain lengths ranging from C₄ to C₃₆, and in particular from C₁₈ to C₃₆, these oils may be linear or branched, saturated or unsaturated; these oils may in particular be heptanoic or octanoic triglycerides, shea oil, alfalfa oil, poppy seed oil, pumpkin oil, millet oil, barley oil, quinoa oil, rye oil, candlenut oil, passionflower oil, shea butter, aloe oil, sweet almond oil, peach kernel oil, groundnut oil, argan oil, avocado oil, baobab oil, borage oil, broccoli oil, calendula oil, camelina oil, canola oil, carrot oil, safflower oil, hemp oil, rapeseed oil, cotton seed oil, coconut oil, marrow seed oil, wheat germ oil, jojoba oil, lily oil, macadamia oil, corn oil, meadowfoam oil, St. John's Wort oil, monoi oil, hazelnut oil, apricot kernel oil, walnut oil, olive oil, evening primrose oil, palm oil, blackcurrant seed oil, kiwi seed oil, grape seed oil, pistachio oil, pumpkin oil, winter squash oil, musk rose oil, sesame oil, soybean oil, sunflower oil, castor oil and watermelon oil, and mixtures thereof, or alternatively caprylic/capric acid triglycerides, such as those sold by STEARINERIE DUBOIS or those sold under the names MIGLYOL 810®, 812® and 818® by DYNAMIT NOBEL,
- linear or branched hydrocarbons of mineral or synthetic origin, such as liquid paraffins and derivatives thereof, petroleum jelly, polydecenes, polybutenes, hydrogenated polyisobutene such as Parleam, squalane;
- synthetic ethers having 10 to 40 carbon atoms such as dicaprylether;
- synthetic esters, such as the oils having the formula R₁COOR₂, wherein R₁ represents a linear or branched fatty acid residue comprising 1 to 40 carbon atoms, and R₂ represents a hydrocarbon chain, particularly branched containing 1 to 40 carbon atoms provided that the sum of the number of carbon atoms of the chains R₁ and R₂ is greater than or equal to 10. The esters may particularly be chosen from fatty alcohol and acid esters, such as for example: cetostearyl octanoate, isopropyl alcohol esters, such as isopropyl myristate, isopropyl palmitate, ethyl palmitate, 2-ethylhexyl palmitate, isopropyl stearate or isostearate, isostearyl isostearate, octyl stearate, hydroxylated esters, such as isostearyl lactate, octyl hydroxystearate, diisopropyl adipate, heptanoates, particularly isostearyl heptanoate, alcohol or polyalcohol octanoates, decanoates or ricinoleates, such as propylene glycol dioctanoate, cetyl octanoate, tridecyl octanoate, 2-ethylhexyl 4-diheptanoate and palmitate, alkyl benzoate, polyethylene glycol diheptanoate, propylene glycol 2-diethylhexanoate, and mixtures thereof, C₁₂-C₁₅ alkyl benzoates, hexyl laurate, neopentanoic acid esters, such as isodecyl neopentanoate, isotridecyl neopentanoate, isostearyl neopentanoate, or octyldodecyl neopentanoate, isononanoic acid esters, such as isononyl isononanoate, isotridecyl isononanoate and octyl isononanoate, hydroxylated esters such as isostearyl lactate and diisostearyl malate;
- polyol esters and pentaerythritol esters, such as dipentaerythrityl tetrahydroxystearate/tetraisostearate;
- esters of diol dimers and diacid dimers, such as Lusplan DD-DA5® and Lusplan DD-DA7®, marketed by NIPPON FINE CHEMICAL and described in the application US 2004-175338;
- copolymers of a diol dimer and of a diacid dimer and esters thereof, such as copolymers of dilinoleyl diol dimers/dilinoleic dimers and esters thereof, for instance Plandool-G;
- copolymers of polyols and of diacid dimers, and esters thereof, such as Hailuscent ISDA, or the copolymer of dilinoleic acid/butanediol;
- fatty alcohols that are liquid at ambient temperature, with a branched and/or unsaturated carbon chain having 12 to 26 carbon atoms, such as 2-octyldodecanol, isostearyl alcohol, oleic alcohol, 2-hexyldecanol, 2-butyloctanol and 2-undecylpentadecanol;
- C₁₂-C₂₂ higher fatty acids, such as oleic acid, linoleic acid, linolenic acid, and mixtures thereof;
- dialkyl carbonates, the two alkyl chains may be identical or different, such as dicaprylyl carbonate marketed under the name CETIOL CC®, by COGNIS;
- oils of higher molar mass having in particular a molar mass ranging from approximately 400 to approximately 10,000 g/mol, in particular from approximately 650 to approximately 10,000 g/mol, in particular from approximately 750 to approximately 7,500 g/mol, and more particularly ranging from approximately 1,000 to approximately 5,000 g/mol. As oils of higher molar mass that can be used in the invention, mention may in particular be made of the oils chosen from:
   - lipophilic polymers,
   - linear fatty acid esters having a total carbon number ranging from 35 to 70,
   - hydroxylated esters,
   - aromatic esters,
   - branched C₂₄-C₂₈ fatty alcohol or fatty acid esters,
   - silicone oils,
   - oils of plant origin,
   - and mixtures thereof;
- fluorinated oils optionally partially hydrocarbon-based and/or silicone-based, such as fluorosilicone oils, fluorinated polyethers or fluorinated silicones, as described in document EP-A-847 752;
- silicone oils, such as polydimethylsiloxanes (PDMS) which are non-volatile and linear or cyclic; polydimethylsiloxanes comprising alkyl, alkoxy or phenyl groups which are pendant or at the end of the silicone chain, said groups having from 2 to 24 carbon atoms such as caprylyl methicone; phenylated silicones, such as phenyl trimethicones, phenyl dimethicones, phenyltrimethylsiloxydiphenylsiloxanes, diphenyl dimethicones, diphenylmethyldiphenyl-trisiloxanes or (2-phenylethyl)trimethylsiloxysilicates, and
- mixtures thereof.

Among the linear or branched hydrocarbons, of mineral or synthetic origin, paraffin oils or petroleum jelly are preferably used.

Among the hydrocarbon oils of plant origin, mention may be made, preferably, of plant oils, such as sweet almond oil, jojoba oil or macadamia nut oil.

According to one embodiment, the compositions according to the invention further comprise at least one surfactant. This surfactant is chosen from the surfactants well known to those skilled in the art.

Preferably, the composition is such that the surfactant is present in a content ranging from 0.5% to 10%, and preferably from 2% to 5%, by weight relative to the total weight of the composition.

These surfactants may be chosen from nonionic, anionic, and amphoteric surfactants, and mixtures thereof. Reference may be made to Kirk-Othmer's Encyclopedia of Chemical Technology, Volume 22, pp. 333-432, 3rd Edition, 1979, Wiley, for the definition of the emulsifying properties and functions of surfactants, in particular pp. 347-377 of this reference, for the anionic, amphoteric and nonionic surfactants.

### Nonionic surfactants

Preferably, the composition according to the invention comprises at least one nonionic surfactant.

The nonionic surfactants may be chosen especially from alkyl and polyalkyl esters of poly(ethylene oxide), oxyalkylenated alcohols, alkyl and polyalkyl ethers of poly(ethylene oxide), optionally polyoxyethylenated alkyl and polyalkyl esters of sorbitan, optionally polyoxyethylenated alkyl and polyalkyl ethers of sorbitan, alkyl and polyalkyl glycosides or polyglycosides, in particular alkyl and polyalkyl glucosides or polyglucosides, alkyl and polyalkyl esters of sucrose, alkyl and polyalkyl esters of glucose, optionally polyoxyethylenated alkyl and polyalkyl esters of glycerol, optionally polyoxyethylenated alkyl and polyalkyl ethers of glycerol, gemini surfactants, silicone surfactant, cetyl alcohol and stearyl alcohol, and mixtures thereof.
1) Alkyl and polyalkyl esters of poly(ethylene oxide) that are preferably used include those with a number of ethylene oxide (EO) units ranging from 2 to 200. Examples that may be mentioned include monostearate 8 EO, whose INCI name is PEG-8 stearate and is sold under the trade name Myrj S8 SO by the company Croda, stearate 40 EO, stearate 50 EO, stearate 100 EO, laurate 20 EO, laurate 40 EO and distearate 150 EO.
2) Alkyl and polyalkyl ethers of poly(ethylene oxide) that are preferably used include those with a number of ethylene oxide (EO) units ranging from 2 to 200. Examples that may be mentioned include cetyl ether 23 EO, oleyl ether 50 EO, phytosterol 30 EO, steareth 40, steareth 100 and beheneth 100.
3) As oxyalkylenated alcohols, which are in particular oxyethylenated and/or oxypropylenated, use is preferably made of those that can comprise from 1 to 150 oxyethylene and/or oxypropylene units, in particular containing from 20 to 100 oxyethylene units, in particular ethoxylated fatty alcohols, especially of C₈-C₂₄ and preferably of C₁₂-C₁₈, which may or may not be ethoxylated, for instance stearyl alcohol ethoxylated with 20 oxyethylene units (CTFA name Steareth-20), for instance Brij 78 sold by the company Uniqema, cetearyl alcohol ethoxylated with 30 oxyethylene units (CTFA name Ceteareth-30), and the mixture of C₁₂-C₁₅ fatty alcohols comprising 7 oxyethylene units (CTFA name C₁₂-C₁₅ Pareth-7), for instance the product sold under the name Neodol 25-7® by Shell Chemicals; or in particular oxyalkylenated (oxyethylenated and/or oxypropylenated) alcohols containing from 1 to 15 oxyethylene and/or oxypropylene units, in particular ethoxylated C₈-C₂₄ and preferably C₁₂-C₁₈ fatty alcohols, such as stearyl alcohol ethoxylated with 2 oxyethylene units (CTFA name Steareth-2), for instance Brij 72 sold by the company Uniqema;
4) Optionally polyoxyethylenated alkyl and polyalkyl esters of sorbitan that are preferably used include those with a number of ethylene oxide (EO) units ranging from 0 to 100. Examples that may be mentioned include sorbitan laurate 4 or 20 EO, in particular polysorbate 20 (or polyoxyethylene (20) sorbitan monolaurate) such as the product Tween 20 sold by the company Uniqema, sorbitan palmitate 20 EO, sorbitan stearate 20 EO, sorbitan oleate 20 EO, or the Cremophor products (RH 40, RH 60, etc.) from BASF. Mention may also be made of the mixture of sorbitan stearate and sucrose cocoate (sold under the name Arlacel 2121U-FL from Croda).
5) Optionally polyoxyethylenated alkyl and polyalkyl ethers of sorbitan that are preferably used include those with a number of ethylene oxide (EO) units ranging from 0 to 100.
6) Alkyl and polyalkyl glucosides or polyglucosides that are preferably used include those containing an alkyl group comprising from 6 to 30 carbon atoms and preferably from 6 to 18 or even from 8 to 16 carbon atoms, and containing a glucoside group preferably comprising from 1 to 5 and especially 1, 2 or 3 glucoside units. The alkylpolyglucosides may be chosen, for example, from decylglucoside (alkyl-C₉/C₁₁-polyglucoside (1.4)), for instance the product sold under the name Mydol 10® by the company Kao Chemicals or the product sold under the name Plantacare 2000 UP® by the company Henkel and the product sold under the name Oramix NS 10® by the company Seppic; caprylyl/capryl glucoside, for instance the product sold under the name Plantacare KE 3711® by the company Cognis or Oramix CG 110® by the company Seppic; laurylglucoside, for instance the product sold under the name Plantacare 1200 UP® by the company Henkel or Plantaren 1200 N® by the company Henkel; cocoglucoside, for instance the product sold under the name Plantacare 818 UP® by the company Henkel; caprylylglucoside, for instance the product sold under the name Plantacare 810 UP® by the company Cognis; the mixture of arachidyl glucoside and behenyl alcohol and arachidyl alcohol, whose INCI name is Arachidyl alcohol (and) behenyl alcohol (and) arachidyl glucoside, sold under the name Montanov 202 by the company Seppic; cetearylglucoside, sold under the trade name Tego care CG 90 by the company Evonik Goldschmidt; methyl glucose sesquistearate sold under the trade name Glucate SS emulsifier by the company Lubrizol, the mixture of C12-C20 alkylglucoside and C14-C22 alcohol (INCI name: C14-C22 alcohol (and) C12-C20 alkylglucoside) sold under the trade name Montanov L by the company Seppic; and mixtures thereof.
7) As alkyl and polyalkyl esters of sucrose, in particular C12-C26 alkyl esters, examples that may be mentioned include sucrose stearate, sold especially under the name Tegosoft PSE 141 G by the company Evonik Goldschmidt, the mixture of sorbitan stearate and sucrose cocoate (sold under the name Arlatone Arlacel 2121 U-FL from Croda), Crodesta F150, the sucrose monolaurate sold under the name Crodesta SL 40, and the products sold by Ryoto Sugar Ester, for instance the sucrose palmitate sold under the references Ryoto Sugar Ester P 1670, Ryoto Sugar Ester LWA 1695 and Ryoto Sugar Ester 01570.
8) Optionally polyoxyethylenated alkyl and polyalkyl esters of glycerol that are preferably used include those with a number of ethylene oxide (EO) units ranging from 0 to 100 and a number of glycerol units ranging from 1 to 30. Examples that may be mentioned include PEG-150 distearate sold under the reference Kessco PEG 6000 DS by the company Italmatch Chemicals Arese, hexaglyceryl monolaurate, the mixture of glyceryl stearate and PEG-100 stearate whose INCI name is glyceryl stearate (and) PEG-100 stearate and that is sold under the trade name Tego Care 180 by the company Evonik Goldscmidt or under the trade name Arlacel 165-FL-(CQ) by the company Croda or under the trade name Simulsol 165 by the company Seppic, the mixture of methylglucose distearate and polyglyceryl (INCI name: polyglyceryl-3 methylglucose distearate) that is sold under the trade name Tego Care 450 by the company Evonik Goldschmidt; citric and stearic ester of polyglycerol (INCI name: polyglyceryl-3 dicitrate/stearate) sold under the trade name Tego Care PSC 3 by the company Evonik Goldschmidt and PEG-30 glyceryl stearate.
9) Optionally polyoxyethylenated alkyl and polyalkyl ethers of glycerol that are preferably used include those with a number of ethylene oxide (EO) units ranging from 0 to 100 and a number of glycerol units ranging from 1 to 30. Examples that may be mentioned include Nikkol batyl alcohol 100 and Nikkol chimyl alcohol 100;
10) cetyl alcohol and stearyl alcohol;
11) Gemini surfactants that may be mentioned include mixture of sodium dilauramidoglutamide lysine and butyleneglycol (INCI name: sodium dilauramidoglutamide lysine) sold under the trade name Pellicer LB -30G by the Company Asahi Kasei Chemicals. The gemini surfactants may also be of formula (I): 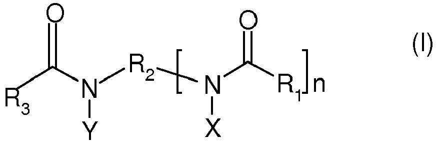 in which:
   - R₁ and R₃ denote, independently of one another, an alkyl radical containing from 1 to 25 carbon atoms;
   - R₂ denotes a spacer consisting of a linear or branched alkylene chain containing from 1 to 12 carbon atoms;
   - X and Y denote, independently of each other, a group -(C₂H₄O)ₐ-(C₃H₆O)_{b}Z, where:
      - Z denotes a hydrogen atom or a radical -CH₂-COOM, -SO₃M, -P(O)(OM)₂, -C₂H₄-SO₃M, -C₃H₆-SO₃M or -CH₂(CHOH)₄CH₂OH, where M and M' represent H or an alkali metal or alkaline-earth metal or ammonium or alkanolammonium ion,
      - a ranges from 0 to 15,
      - b ranges from 0 to 10, and
      - the sum of a + b ranges from 1 to 25; and
   - n ranges from 1 to 10.

The gemini surfactant of formula (I) is preferably such that each of the groups R₁-CO- and R₃-CO- comprises from 8 to 20 carbon atoms, and preferably denotes a coconut fatty acid residue (comprising mainly lauric acid and myristic acid).

In addition, this surfactant is preferably such that, for each of the X and Y radicals, the sum of a and b has an average value ranging from 10 to 20 and is preferably equal to 15. A preferred group for Z is the group -SO₃M, where M is preferably an alkali metal ion such as a sodium ion.

The spacer R₂ advantageously consists of a linear C₁-C₃ alkylene chain, and preferably an ethylene (CH₂CH₂) chain.

Finally, n is advantageously equal to 1.

A surfactant of this type is in particular the one identified by the INCI name: Sodium dicocoylethylenediamine PEG-15 sulfate, having the following structure: 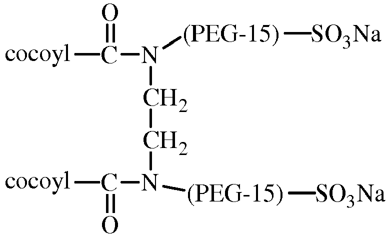 it being understood that PEG represents the CH₂CH₂O group and cocoyl represents the coconut fatty acid residue.

This surfactant has a molecular structure very similar to that of ceramide-3.

Preferably, the gemini surfactant according to the invention is used as a mixture with other surfactants, and in particular as a mixture with (a) an ester of a C₆-C₂₂ fatty acid (preferably C₁₄-C₂₀ such as a stearate) and of glyceryl, (b) a diester of a C₆-C₂₂ fatty acid (preferably C₁₄-C₂₀ such as a stearate) and of citric acid and of glycerol (in particular a diester of a C₆-C₂₂ fatty acid and of glyceryl monocitrate), and (c) a C₁₀-C₃₀ fatty alcohol (preferably behenyl alcohol).

Advantageously, the composition according to the invention comprises a mixture of sodium dicocoylethylenediamine PEG-15 sulfate, of glyceryl stearate, of glyceryl stearate monocitrate, of behenyl alcohol.

More preferentially, the gemini surfactant according to the invention represents from 10 to 20% by weight, and advantageously 15% by weight; the ester of a C₆-C₂₂ fatty acid and of glyceryl represents from 30 to 40% by weight, advantageously 35% by weight; the diester of a C₆-C₂₂ fatty acid and of citric acid and of glycerol represents from 10 to 20% by weight, advantageously 15% by weight; and the C₁₀-C₃₀ fatty alcohol represents from 30 to 40% by weight, advantageously 35% by weight, relative to the total weight of the mixture of surfactants containing the gemini surfactant.

Advantageously, the composition according to the invention comprises a mixture of from 10 to 20% by weight of sodium dicocoylethylenediamine PEG-15 sulfate, from 30 to 40% (in particular 35%) by weight of glyceryl stearate, from 10 to 20% (in particular 15%) by weight of glyceryl stearate monocitrate and from 30 to 40% (in particular 35%) by weight of behenyl alcohol, relative to the total weight of the mixture of surfactants comprising the gemini surfactant.

The gemini surfactant may be used, for example, as a mixture with other surfactants in the form of the products sold by Sasol under the Ceralution® names, in particular the following products:
- Ceralution® H: Behenyl Alcohol, Glyceryl Stearate, Glyceryl Stearate Citrate and Sodium Dicocoylethylenediamine PEG-15 Sulfate,
- Ceralution® F: Sodium Lauroyl Lactylate and Sodium Dicocoylethylenediamine PEG-15 Sulfate,
- Ceralution® C: Aqua, Capric/Caprylic triglyceride, Glycerin, Ceteareth-25, Sodium Dicocoylethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben (INCI names).

This gemini surfactant represents from 3 to 50% of the weight of these mixtures.

Preferably, the composition comprises as gemini surfactant the compound whose INCI name is Behenyl alcohol, glyceryl stearate, glyceryl stearate citrate and sodium dicocoylethylenediamine PEG-15 sulfate, sold under the name Ceralution® H by the company Sasol.
12) The term "silicone surfactant" is intended to mean a silicone compound comprising at least one oxyalkylenated chain, in particular comprising at least one oxyethylenated (-OCH₂CH₂-) and/or oxypropylenated (-OCH₂CH₂CH₂-) chain and/or polyglycerol chain. As silicone surfactant, examples that may be mentioned include polydimethylsiloxanes comprising both oxyethylenated groups and oxypropylenated groups. Mention may, for example, be made of the polydimethylsiloxane with an oxyethylene/oxypropylene ending sold as a mixture with caprylic/capric acid triglycerides under the name Abil care 85 by the company Evonik Goldschmidt (INCI name: BIS-PEG/PPG-16/16 PEG/PPG-16/16 Dimethicone/Caprylic/Capric Triglyceride), the polydimethylsiloxane comprising an alpha-omega polyether group (OE/OP: 40/60), sold under the name Abil B8832 by the company Goldschmidt (INCI name: BIS-PEG/PPG-20/20 Dimethicone), the oxyethylenated oxypropylenated polydimethylsiloxane sold under the name Abil B88184 by the company Goldschmidt (INCI name: PEG/PPG-20/6 Dimethicone), the polydimethylsiloxane with oxyethylene/oxypropylene ending, sold under the name Abil Care XL 80 by the company Evonik Goldscmidt (INCI name: BIS-PEG/PPG-20/5 PEG/PPG20/5 Dimethicone; Methoxy PEG/PPG-25/4) Dimethicone; Caprylic/Capric Triglyceride and the oxyethylenated oxypropylenated polydimethyl/methylsiloxane sold under the name Abil B8852 by the company Goldschmidt (INCI name: PEG/PPG-4/12 Dimethicone), and mixtures thereof. Alternatively, silicone surfactant may also be a polydimethylsiloxane with polyglyceryl chains, such as polyglyceryl-3 polymethylsiloxyethyl dimethicone, laurylpolyglyceryl-3 polymethylsiloxyethyl dimethicone and polyglyceryl-3 disiloxane dimethicone and mixtures thereof. Such silicone surfactants are described in patent application EP 1 213 316. According to a particular embodiment, the silicone surfactant is a polyglyceryl-3 disiloxane dimethicone sold under the trade name KF 6100 by the company Shin Etsu.
13) and mixtures thereof.

In a preferred embodiment, the nonionic surfactant is a silicone surfactant. Preferably, the silicone surfactant is polydimethylsiloxane with an oxyethylene/oxypropylene ending, such as the one sold as a mixture with caprylic/capric acid triglycerides under the name Abil care 85 by the company Evonik Goldschmidt (INCI name: BIS-PEG/PPG-16/16 PEG/PPG-16/16 Dimethicone/Caprylic/Capric Triglyceride).

In another preferred embodiment, the nonionic surfactant is chosen from polyoxyethylenated alkyl and polyalkyl ester of glycerol. Preferably, the polyalkyl ester of glycerol is citric and stearic ester of polyglycerol (INCI name: polyglyceryl-3 dicitrate/stearate) sold under the trade name Tego Care PSC 3 by the company Evonik Goldschmidt.

In another preferred embodiment, the nonionic surfactant is a gemini surfactant of formula (I) as defined above. Preferably, the gemini surfactant is identified by the INCI name: Sodium dicocoylethylenediamine PEG-15 sulfate, having the above-mentioned structure.

Preferably, the composition comprises as gemini surfactant the compound whose INCI name is Behenyl alcohol, glyceryl stearate, glyceryl stearate citrate and sodium dicocoylethylenediamine PEG-15 sulfate, sold under the name Ceralution® H by the company Sasol.

### Anionic surfactants

In another embodiment, the composition according to the invention comprises at least one anionic surfactant. As anionic surfactants may be mentioned carboxylates (sodium 2-(2-hydroxyalkyloxy)acetate), amino acid derivatives (N-acylglutamates, N-acylglycinates or acylsarcosinates), alkyl sulfates, alkyl ether sulfates and oxyethylenated derivatives thereof, sulfonates, isethionates and N-acylisethionates, taurates and N-acyl N-methyltaurates, sulfosuccinates, alkyl sulfoacetates, phosphates and alkyl phosphates, polypeptides, anionic derivatives of alkyl polyglycoside (acyl-D-galactoside uronate), and fatty acid soaps, and mixtures thereof.

The phosphate surfactant may be selected from monoalkyl phosphates, dialkyl phosphates, salts of monoalkyl phosphates, salts of dialkyl phosphates, and mixtures thereof. More preferably, the monoalkyl phosphates and dialkyl phosphates comprise one or more linear or branched and aliphatic and/or aromatic alkyl chains having from 8 to 22 carbon atoms. According to preferred embodiments, the phosphate surfactant(s) can be neutralized with organic or inorganic bases such as, for example, potassium hydroxide, sodium hydroxide, triethanolamine, arginine, lysine and N-methylglucamine to form the aforementioned salts.

Suitable examples of phosphate surfactants include, but are not limited to, monolauryl phosphate, such as the product sold under the name MAP 20® by Kao Chemicals, the potassium salt of dodecyl phosphate, such as the mixture of mono- and diester (predominantly diester) sold under the name Crafol AP-31® by Cognis, the octyl monoester and the octyl diester of phosphoric acid, such as the mixture sold under the name Crafol AP-20® by Cognis, the ethoxylated (7 mol. of EO) 2-butyloctanol monoester and the ethoxylated (7 mol. of EO) 2-butyloctanol diester of phosphoric acid, such as the mixture sold under the name Isofol 12 7 EO-Phosphate Ester® by Condea, the potassium or triethanolamine salts of monoalkyl (C₁₂-C₁₃) phosphate, such as the product sold under the references Arlatone MAP 230K-40® and Arlatone MAP 230T-60® by Uniqema, potassium lauryl phosphate, such as the product as a 40% aqueous solution sold under the name Dermalcare MAP XC99/09® by Rhodia Chimie, potassium cetyl phosphate, such as the product sold under the name Arlatone MAP 160K® by Uniqema or under the trade name Amphisol K by DSM Nutritional Products, and the mixtures of these surfactants.

### Amphoteric surfactants

In another embodiment, the composition according to the invention comprises at least one amphoteric surfactant. As amphoteric surfactant may be mentioned betaines, N-alkylamidobetaines and derivatives thereof, glycine derivatives, sultaines, alkyl polyaminocarboxylates and alkyl amphoacetates, and mixtures thereof.

### Additional dyes

The compositions according to the invention may also comprise a dye or a mixture of dyes, other than the coated pigments as defined above.

According to one embodiment, the additional dye content is 0.1% to 20%, preferably 0.5% to 15%, and more preferably 1% to 10%, by weight in relation to the total weight of said composition.

The additional dye (or coloring agent) present in the compositions according to the invention is chosen for example from the group consisting of uncoated pigments (other than those defined above), colorants, nacres and reflective particles (or glitter).

A cosmetic composition according to the invention may advantageously incorporate at least one dye selected from organic or inorganic dyes, in particular such as pigments or nacres conventionally used in cosmetic compositions, liposoluble or water-soluble colorants, materials with a specific optical effect, and mixtures thereof.

According to one embodiment, the dye is chosen from uncoated pigments. In this way, the compositions according to the invention may also further comprise at least one uncoated pigment.

According to one embodiment, the uncoated pigment content is 0.1% to 20%, preferably 0.5% to 15%, and more preferably 1% to 10%, by weight in relation to the total weight of said composition.

Examples of inorganic pigments, organic pigments and nacres have been described above.

According to one embodiment, the additional dye is chosen from colorants.

The term "colorants" refers to generally organic compounds soluble in fats such as oils or in a hydroalcoholic phase.

The cosmetic composition according to the invention may also comprise water-soluble or liposoluble colorants. The liposoluble colorants are, for example, Sudan Red, DC Red 17, DC Green 6, β-carotene, soybean oil, Sudan Brown, DC Yellow 11, DC Violet 2, DC Orange 5, and Quinoline Yellow. The water-soluble colorants are, for example, beetroot juice and caramel.

The compositions according to the invention may also contain at least one material with a specific optical effect, also referred to as glitter or reflective particles.

This effect is different from a simple conventional hue effect, i.e. a unified and stabilized effect of the kind produced by conventional dyes, such as, for example, monochromatic pigments. For the purpose of the invention, the term "stabilized" signifies absence of an effect of variability of color with the angle of observation or in response to a temperature change.

For example, this material may be selected from particles having a metallic glint, goniochromatic coloring agents, diffracting pigments, thermochromatic agents, optical brighteners, and also fibers, in particular of the interference type. Of course, these various materials may be combined so as to provide the simultaneous manifestation of two effects, or even a new effect in accordance with the invention.

The metallic-glint particles that can be used in the invention are in particular chosen from:
- particles of at least one metal and/or of at least one metal derivative,
- particles comprising a single-substance or multi-substance, organic or mineral substrate, at least partially coated with at least one metallic-glint layer comprising at least one metal and/or at least one metal derivative, and
- mixtures of said particles.

Among the metals that may be present in said particles, mention may, for example, be made of Ag, Au, Cu, Al, Ni, Sn, Mg, Cr, Mo, Ti, Zr, Pt, Va, Rb, W, Zn, Ge, Te, Se and mixtures or alloys thereof. Ag, Au, Cu, Al, Zn, Ni, Mo, Cr and mixtures or alloys thereof (for example, bronzes and brasses) are preferred metals.

The term "metal derivatives" denotes compounds derived from metals, in particular oxides, fluorides, chlorides and sulfides.

### Fillers

The compositions according to the invention may also comprise at least one filler, of organic or mineral nature, which makes it possible in particular to confer thereon additional properties of enhanced matteness, covering power, staying power and/or stability.

The term "filler" should be understood to mean colorless or white solid particles of any shape, which are in a form that is insoluble or dispersed in the medium of the composition. Mineral or organic in nature, they make it possible to confer body or rigidity on the composition, and/or softness, and uniformity on the makeup.

The fillers used in the compositions according to the present invention may be of lamellar, globular or spherical form, or in the form of fibers or in any other intermediate form between these defined forms.

The fillers may or may not be coated superficially, and in particular they may be surface-treated with silicones, amino acids, fluorinated derivatives or any other substance that promotes the dispersion and compatibility of the filler in the composition.

As examples of mineral fillers, mention may be made of talc, mica, silica, hollow silica microspheres, kaolin, calcium carbonate, magnesium carbonate, hydroxyapatite, boron nitride, glass or ceramic microcapsules, composites of silica and of titanium dioxide, such as the TSG series marketed by Nippon Sheet Glass.

As examples of organic fillers, mention may be made of polyamide powder (Nylon® Orgasol from Atochem), polyethylene powder, polymethyl methacrylate powder, polytetrafluoroethylene powder (Teflon), acrylic acid copolymer powder (Polytrap from Dow Corning), lauroyl lysine, hollow polymeric microspheres such as those of polyvinylidene/acrylonitrile chloride such as Expancel (Nobel Industries), hexamethylene diisocyanate/Trimethylol hexyllactone copolymer powder (Plastic Powder from Toshiki), silicone resin microbeads (Tospearl from Toshiba for example), synthetic or natural micronized waxes, metal soaps derived from organic carboxylic acids having 8 to 22 carbon atoms, and preferably from 12 to 18 carbon atoms, for example, zinc stearate, magnesium stearate or lithium stearate, zinc laurate, magnesium myristate, Polypore® L 200 (Chemdal Corporation), cross-linked elastomeric organopolysiloxane powders coated with silicone resin, in particular silsesquioxane resin, as described for example in the patent US 5 538 793, polyurethane powders, in particular, cross-linked polyurethane powders including a copolymer, said copolymer comprising trimethylol hexyllactone. In particular, it may be a polymer of hexamethylene diisocyanate/trimethylol hexyllactone. Such particles are in particular commercially available, for example under the name PLASTIC POWDER D-400® or PLASTIC POWDER D-800® from TOSHIKI, and mixtures thereof.

According to one particular embodiment of the invention, the composition comprises at least one cross-linked elastomeric organopolysiloxane powder.

The elastomeric organopolysiloxane powder(s) may be present at a content ranging from 0.5% to 12% by weight, advantageously from 1% to 8% by weight in relation to the total weight of said composition.

In particular, mention may be made of cross-linked elastomeric organopolysiloxane powders coated with silicone resin, in particular silsesquioxane resin, as described for example in the patent US 5 538 793. Such elastomer powders are sold under the names KSP-100®, KSP-101®, KSP-102®, KSP-103®, KSP-104® and KSP-105® by SHIN ETSU; mention may also be made of cross-linked elastomeric organopolysiloxane powders coated with silicone resin such as hybrid silicone powders functionalized by fluoroalkyl groups, in particular sold under the name "KSP-200" by Shin Etsu; or hybrid silicone powders functionalized by phenyl groups, in particular sold under the name "KSP-300" by Shin Etsu.

According to one advantageous embodiment, the compositions according to the invention comprise fillers chosen from silicone fillers, particularly KSP-100® and KSP-300®.

### Additives

A cosmetic composition according to the invention may also further comprise any additive normally used in the field in question, for example chosen from gums, resins, dispersants, polymers, antioxidants, essential oils, preservatives, fragrances, neutralizing agents, antiseptic agents, anti-UV protective agents, cosmetic active agents, such as vitamins, hydrating agents, emollients or collagen-protecting agents, and mixtures thereof.

A person skilled in the art can adjust the type and amount of additives present in the compositions according to the invention by means of routine operations, so that the cosmetic properties and the stability properties sought for these compositions are not affected by the additives.

### Applications

The cosmetic compositions covered by the invention may be face or body treatment or makeup products.

The compositions according to the invention are cosmetic compositions intended for makeup and/or skincare.

Preferably, the compositions according to the invention are in the form of a foundation.

These compositions are thus intended to be applied onto the skin.

The present invention also relates to a non-therapeutic cosmetic skin treatment method comprising a step for applying at least one layer of a composition according to the invention onto the skin.

The present invention also relates to a non-therapeutic makeup and/or skincare method comprising a step for applying at least one layer of a composition as defined above onto the skin.

The present invention also relates to a skin makeup method wherein a composition as defined above is applied.

Throughout the application, the term "comprising a" or "including a" means "comprising at least one" or "including at least one", unless otherwise specified.

Throughout the above description, unless specified otherwise, the term "between x and y" refers to an inclusive range, i.e. the values x and y are included in the range.

The invention will now be illustrated in the following non-limiting examples. Unless specified otherwise, the % are expressed by weight in relation to the total weight of the composition.

The compositions are prepared using routine cosmetic composition formulation methods.

### EXAMPLES

The various compositions were evaluated in respect of the appearance thereof, cosmetics thereof, particularly the application property thereof. A composition is thus acceptable according to the invention if it exhibits a satisfactory macroscopic and microscopic appearance, and enhanced cosmetics compared to the formula without coated pigment.

### Influence of associative polyurethane structure

The three compositions below are prepared and tested according to the protocols described hereinafter.

| Phase | Chemical name | 1 | 2 (non-invention) | 3 (non-invention) |
|---|---|---|---|---|
| A1 | Polyethylene glycol mono-stearate (8 OE) | 1.3 | 1.3 | 1.3 |
| | Stearic acid | 0.3 | 0.3 | 0.3 |
| | Stearyl alcohol | 0.5 | 0.5 | 0.5 |
| | Cyclohexadimethicone | 18 | 18 | 18 |
| A2 | Iron oxides and titanium oxides coated with Disodium Stearoyl Glutamate & Aluminum Hydroxide (=NAl) | 10 | 10 | 10 |
| | Cyclohexadimethicone | 5 | 5 | 5 |
| B1 | Water | 30.95 | 30.95 | 30.95 |
| | Xanthan gum | 0.35% | 0.35% | 0.35% |
| B2 | Glycerin | 7 | 7 | 7 |
| | Preservatives | 0.9 | 0.9 | 0.9 |
| | Sucrose mono-di-palmito-stearate | 1.3 | 1.3 | 1.3 |
| B3 | Water | qs 100 | qs 100 | qs 100 |
| | Steareth-100/PEG-136/HDI copolymer (RHEOLATE FX-1100) | 1.5 | - | - |
| | PEG-240/HDI COPOLYMER BIS-DECYLTETRADECETH-20 ETHER (Adekanol GT-700) | - | 1.5 | - |
| | Bis-C12-14 Pareth-3/C16-20 Pareth-11 HDI/PEG-130 Copolymer containing 20% active substance (AS) in water (Polyurethane-39, Luvigel star) | - | - | 7.5 (i.e. 1.5% AS) |
| C | FILLER (Cross-linked PDMS gum beads - KSP 300) | 2 | 2 | 2 |

Composition 1 consists of a composition according to the invention comprising a polyurethane according to formula (I) and a pigment coated with a lipophilic compound.
Compositions 2 and 3 (not according to the invention) are compositions comprising an associative polyurethane not complying with formula (I) according to the invention. Indeed, these compositions comprise a polyurethane with a branched hydrophobic end and a PEG chain length at the ends of less than 40.

### Composition preparation protocol

Phase A1 was heated to 70°C. A2 was then introduced into phase A1 under Raynerie stirring and maintained at 65°C. B1 was then prepared with a Moritz by sprinkling the hydrophilic gelling agent in water and the whole was left under stirring for 30 minutes and B2 was then added. Mixture B1-B2 has heated to 70°C and maintained at 65-66°C

EMULSION: again at 65°C, A1+A2 was incorporated in B1+B2 with a Moritz and stirred for 10 minutes then allowed to cool gently (using a cold water bath if required), to T=45-50°C. With a Raynerie, B3 was added for 10min. Cooling was continued to 35°C and phase C was added.

### Composition evaluation protocol

- Appearance of formula: The macroscopic appearance of the formula was observed particularly in respect of the homogeneity of the formula and more particularly if it involves salting out or not, the surface condition thereof and the fluidity thereof. The microscopic appearance was also observed using an optical microscope particularly in respect of the regularity of the base of the emulsion, the fineness, the condition of the edges (sharp or with splitting). A score of + to ++++ was given by combining all these criteria. Only the scores +++ and ++++ are considered to be acceptable according to the invention.
- Cosmetic application properties: Each composition was applied onto the face at a rate of 0.2 g of composition per face. The cosmetic properties evaluated are particularly the application properties: Satisfactory adherence, slip, application time. A score of + to ++++ was given by combining all these criteria. Only the scores +++ and ++++ are considered to be acceptable according to the invention.

| | Example 1 | Example 2 (Non-invention) | Example 3 (Non-invention) |
|---|---|---|---|
| Appearance of formula | ++++ Macroscopic appearance: Moderately thick, homogeneous, smooth and glossy formula | + Macroscopic appearance: Non-homogeneous formula Microscopic appearance: Coarse and irregular appearance | ++ Macroscopic appearance: Relatively fluid, homogeneous formula |
| | Microscopic appearance: Relatively fine, regular emulsion with sharp edges | | Microscopic appearance: Very coarse emulsion with splitting |
| Application of formula | +++ Satisfactory application, slight lack of adhesion | +++ Satisfactory application, slight lack of adhesion | ++ Mediocre application, less satisfactory adhesion |

The appearance of the formulas obtained with the compositions from examples 2 and 3 (non-invention) is not homogeneous.

The associative polyurethane according to the invention is the only one to result in a composition (example 1 according to the invention) which is macroscopically and microscopically homogeneous.

### Influence of pigment coating type

| Phase | Chemical name | 4 | 5 | 6 | 7 | 8 (non-invention) |
|---|---|---|---|---|---|---|
| A1 | Polyethylene glycol mono-stearate (8 OE) | 1.3 | 1.3 | 1.3 | 1.3 | 1.3 |
| | Stearic acid | 0.3 | 0.3 | 0.3 | 0.3 | 0.3 |
| | Stearyl alcohol | 0.5 | 0.5 | 0.5 | 0.5 | 0.5 |
| | Cyclohexadimethicone | 13 | 13 | 13 | 13 | 13 |
| A2 | Iron oxides and titanium oxides coated with Disodium Stearoyl Glutamate & Aluminum Hydroxide (=NAl) | 10 | | | | |
| | Iron oxides and titanium oxide coated with Dimethicone (=SA) | | 10 | | | |
| | Iron oxides and titanium oxide coated with Triethoxycaprylylsilane (=AS) | | | 10 | | |
| | Iron oxides and titanium oxide coated with Hydrogenated Lecithin (=HLC) | | | | 10 | |
| | Uncoated iron oxides and titanium oxide (Sunpuro) | | | | | 10 |
| | Cyclohexadimethicone | 5 | 5 | 5 | 5 | 5 |
| B1 | Water | qs 100 | qs 100 | qs 100 | qs 100 | qs 100 |
| | Glycerin | 7 | 7 | 7 | 7 | 7 |
| | Preservatives | 0.9 | 0.9 | 0.9 | 0.9 | 0.9 |
| | Sucrose mono-di-palmito-stearate | 1.3 | 1.3 | 1.3 | 1.3 | 1.3 |
| B2 | Cyclohexadimethicone | 5 | 5 | 5 | 5 | 5 |
| | Steareth-100/PEG-136/HDI copolymer (RHEOLATE FX-1100) | 1.5 | 1.5 | 1.5 | 1.5 | 1.5 |
| C | FILLER (Cross-linked PDMS gum beads - KSP 300) | 2 | 2 | 2 | 2 | 2 |

### Composition preparation protocol

Phase A1 was heated to 70°C. A2 was introduced into phase A1 under Raynerie stirring and maintained at 65°C. Phase B1 was prepared and was heated to 70°C and maintained at 65-66°C.

EMULSION: again at 65°C, A1+A2 was incorporated in B1 with a Moritz and stirred for 10 minutes then allowed to cool gently (using a cold water bath if required), to T=45-50°C. With a Raynerie, B2 was added for 10min. Cooling was continued to 35°C and phase C was added.

| | Appearance of formula | Application of formula |
|---|---|---|
| Example 4 (NAl pigments) | ++++ Macroscopic appearance: Homogeneous, smooth and glossy formula | ++++ Very good application, satisfactory adhesion, satisfactory slip, satisfactory application time |
| | Microscopic appearance: Relatively fine, regular emulsion with sharp edges | |
| Example 5 (SA pigments) | +++ Macroscopic appearance: Homogeneous, smooth and glossy formula | ++++ Very good application, satisfactory adhesion, satisfactory slip, satisfactory application time |
| | Microscopic appearance: Moderately fine, regular emulsion with sharp edges | |
| Example 6 (AS pigments) | ++++ Macroscopic appearance: Homogeneous, smooth and glossy formula | +++ Satisfactory application, acceptable adhesion, satisfactory slip, satisfactory application time |
| | Microscopic appearance: Fine, regular emulsion with sharp edges | |
| Example 7 (HLC pigments) | ++++ Macroscopic appearance: Homogeneous, smooth and glossy formula | ++++ Very good application, satisfactory adhesion, satisfactory slip, satisfactory application time |
| | Microscopic appearance: Relatively fine, regular emulsion with sharp edges | |
| Example 8 (Uncoated pigments) | ++++ Macroscopic appearance: Homogeneous, smooth and glossy formula | ++ Mediocre adhesion, the formula is more difficult to spread and has a relatively short application time. |
| | Microscopic appearance: Relatively fine, regular emulsion with sharp edges | |

Adding a hydrophobic coated pigment makes it possible to enhance the application qualities of the composition, compared to example 8 (non-invention) containing hydrophobic uncoated pigments.

### Influence of hydrophilic gelling agent type

The following compositions are prepared according to the protocol described hereinafter and tested according to the protocols described below.

They were prepared to study the influence of the presence of an hydrophilic gelling agent and also the influence of the nature of said agent.

| Phase | Chemical name | 4 | 1 | 9 | 10 | 11 |
|---|---|---|---|---|---|---|
| A1 | Polyethylene glycol mono-stearate (8 OE) | 1.3 | 1.3 | 1.3 | 1.3 | 1.3 |
| | Stearic acid | 0.3 | 0.3 | 0.3 | 0.3 | 0.3 |
| | Stearyl alcohol | 0.5 | 0.5 | 0.5 | 0.5 | 0.5 |
| | Cyclohexadimethicone | 13 | 13 | 13 | 13 | 13 |
| A2 | Pigments coated with Disodium Stearoyl Glutamate & Aluminum Hydroxide (=NAl) | 10 | 10 | 10 | 10 | 10 |
| | Cyclohexadimethicone | 5 | 5 | 5 | 5 | 5 |
| B1 | Water | qs 100 | qs 100 | qs 100 | qs 100 | qs 100 |
| | Xanthan gum | | 0.35% | - | - | - |
| | ACRYLAMIDE/SODIUM ACRYLOYLDIMETHYLTAURATE COPOLYMER (and) ISOHEXADECANE (and) POLYSORBATE 80 - Simulgel 600 containing 40% active substance | | - | 0.3% | - | - |
| | Scleroglucan gum - Amigum | | - | - | 0.2% | - |
| | AMMONIUM POLYACRYLOYLDIMETHYL TAURATE - Hostacerin AMPS containing 97% active substance | | - | - | - | 0.2% |
| | Poly vinyl alcohol - SELVOL 540 | | | | | |
| B2 | Glycerin | 7 | 7 | 7 | 7 | 7 |
| | Preservatives | 0.9 | 0.9 | 0.9 | 0.9 | 0.9 |
| | Sucrose mono-di-palmito-stearate | 1.3 | 1.3 | 1.3 | 1.3 | 1.3 |
| B3 | Cyclohexadimethicone | 5 | 5 | 5 | 5 | 5 |
| | Steareth-100/PEG-136/HDI copolymer (RHEOLATE FX-1100) | 1.5 | 1.5 | 1.5 | 1.5 | 1.5 |
| C | FILLER (Cross-linked PDMS gum beads - KSP 300) | 2 | 2 | 2 | 2 | 2 |

The hydrophilic gelling agent content is adapted to obtain equivalent gelling.

### Composition preparation protocol

Heat phase A1 to 70°C. Introduce A2 into phase A1 under Raynerie stirring and maintain at 65°C - Prepare B1 with a Moritz by sprinkling the hydrophilic gelling agent in water and leave to stir for 30 minutes and then add B2. Heat the mixture B1-B2 to 70°C and maintain at 65-66°C

EMULSION: again at 65°C, incorporate A1+A2 in B1+B2 with a Moritz and stir for 10 minutes then allow to cool gently (using a cold water bath if required), to T=45-50°C. With a Raynerie, add B3 (increase speed if required) for 10min. Continue cooling to 35°C and add phase C.

Examples 1, 9, 10, and 11 have shown that adding an hydrophilic gelling agent makes it possible to enhance the two months stability of the compositions at 45°C, without however degrading the application qualities thereof.

### Influence of surfactant type

The four compositions below are prepared and tested according to the protocols described hereinafter.

| Phase | Chemical name | 12 | 13 | 14 | 15 |
|---|---|---|---|---|---|
| A1 | PEG-8 stearate (MYRJ S8-SO-(MV) sold by Croda) | 2 | - | - | - |
| | BIS-PEG/PPG-16/16 PEG/PPG-16/16 Dimethicone/Caprylic/Capric Triglyceride (Abil Care 85, sold by Evonik Goldschmidt) | - | 2 | - | - |
| | Behenyl Alcohol, Glyceryl Stearate, Glyceryl Stearate Citrate and Sodium Dicocoylethylenediamine PEG-15 Sulfate (Ceralution H sold by Sasol) | - | - | 2 | - |
| | polyglyceryl-3 dicitrate/stearate (Tego Care PSC 3 sold by Evonik Goldschmidt) | - | - | - | 2 |
| | Stearic acid | 0.3 | 0.3 | 0.3 | 0.3 |
| | Stearilic alcohol | 0.5 | 0.5 | 0.5 | 0.5 |
| | Caprylyl methicone | 18 | 18 | 18 | 18 |
| A2 | Iron and titanium oxides coated with disodium stearoyl glutamate & aluminum hydroxyde (=NAl) | 10 | 10 | 10 | 10 |
| | Caprylyl methicone | 5 | 5 | 5 | 5 |
| B1 | Water | qsp 100 | qsp 100 | qsp 100 | qsp 100 |
| | Xanthan gum | 0.35% | 0.35% | 0.35% | 0.35% |
| B2 | Glycerin | 7 | 7 | 7 | 7 |
| | Preservatives | 0.9 | 0.9 | 0.9 | 0.9 |
| | Sucrose mono-di-palmito-stearate | 1.5 | 1.5 | 1.5 | 1.5 |
| B3 | Water | 20.9 | 20.9 | 20.9 | 20.9 |
| | Steareth-100/PEG-136/HDI copolymer (RHEOLATE FX 1100 sold by Elementis) | 1.5 | 1.5 | 1.5 | 1.5 |
| C | Filler (reticulated PDMS gum-KSP 300) | 2 | 2 | 2 | 2 |

The compositions are prepared and evaluated as disclosed in the previous examples.

### Evaluation of the compositions

The stability of the compositions of example 12 to 15 is evaluated by macroscopic and microscopic observation after 24 hours, one month and two months at ambient temperature, 45°C.

The composition must not exhibit modifications in macroscopic appearance; it must remain smooth and homogeneous, without release, without phase separation and without change in colour.

The composition is observed under a microscope between microscope slide and coverglass at a magnification x10. Its microscopic appearance must remain close to the initial aspect: in particular, decomposition of the emulsion must not be observed (emulsion base coarser, coalescence reflected by the presence of numerous large drops, modification of the preparation edges, presence of crystals).

The test is regarded as stable if the emulsion remains fine, without release over the edges and without coalescence.

The compositions of examples 13 to 15 showed an improved stability in comparison to the composition of example 12.

## Claims

1. Cosmetic composition comprising a physiologically acceptable medium, in the form of an oil-in-water emulsion, containing:
- at least one associative polyurethane complying with the following formula (I): 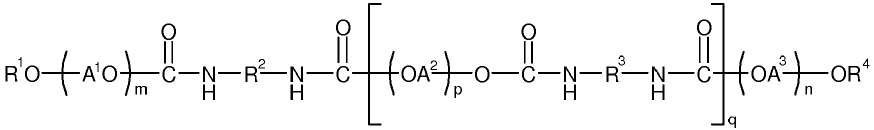 in which:
* R¹ and R⁴ represent, independently of each other, a linear hydrocarbon radical comprising 1 to 30 carbon atoms,
* A¹, A² and A³ represent, independently of each other, a linear or branched alkylene radical, having 2 to 4 carbon atoms;
* m and n represent, independently of each other, an integer between 35 and 500,
* p represents an integer between 5 and 500,
* q represents an integer between 1 and 8,
* R² and R³ represent, independently of each other, a linear or branched bivalent hydrocarbon radical, comprising 1 to 30 carbon atoms,
- and pigment particles coated with at least one lipophilic compound, the average size of said particles being greater than 100 nm,
and wherein the pigment particles are in the oil phase of said emulsion, and wherein the coated pigment particles are iron oxide and/or titanium oxide particles coated with a lipophilic compound chosen from the group consisting of disodium stearoyl glutamate, isopropyl trisostearyl titanate, dimethicone, triethoxy caprylylsilane, hydrogenated lecithin and mixtures thereof.

2. Cosmetic composition according to claim 1, comprising 0.01% to 10% by weight of associative polyurethane active substance having formula (I) in relation to the total weight of said composition.

3. Cosmetic composition according to claim 1 or 2, wherein, in formula (I), A¹, A² and A³ represent an ethylene radical.

4. Cosmetic composition according to any of claims 1 to 3, wherein, in formula (I), R¹ and R⁴ represent, independently of each other, a linear alkyl group comprising 8 to 30 carbon atoms.

5. Composition according to claim 4, wherein, in formula (I), R¹ and R⁴ represent a linear alkyl group comprising 18 carbon atoms.

6. Cosmetic composition according to any of claims 1 to 5, wherein, in formula (I), m and n represent, independently of each other, an integer between 50 and 200.

7. Cosmetic composition according to any of claims 1 to 6, comprising 0.1% to 30% by weight of pigment particles coated with at least one lipophilic compound in relation to the total weight of said composition.

8. Cosmetic composition according to any of claims 1 to 7, comprising at least one surfactant.

9. Cosmetic composition according to any of claims 1 to 8, comprising at least one non ionic surfactant.

10. Cosmetic composition according to claim 9, wherein the non ionic surfactant is a silicone surfactant, in particular polydimethylsiloxane with an oxyethylene/oxypropylene ending.

11. Cosmetic composition according to claim 9, wherein the non ionic surfactant is a polyoxyethylenated alkyl and polyalkyl ester of glycerol, in particular citric and stearic ester of polyglycerol.

12. Cosmetic composition according to claim 9, wherein the non ionic surfactant is a gemini surfactant, in particular of formula (I): 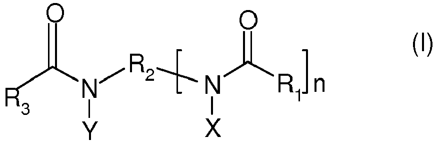 in which:
- R₁ and R₃ denote, independently of one another, an alkyl radical containing from 1 to 25 carbon atoms;
- R₂ denotes a spacer consisting of a linear or branched alkylene chain containing from 1 to 12 carbon atoms;
- X and Y denote, independently of each other, a group -(C₂H₄O)ₐ-(C₃H₆O)_{b}Z, where:
• Z denotes a hydrogen atom or a radical -CH₂-COOM, -SO₃M,-P(O)(OM)₂, -C₂H₄-SO₃M, -C₃H₆-SO₃M or -CH₂(CHOH)₄CH₂OH, where M and M' represent H or an alkali metal or alkaline-earth metal or ammonium or alkanolammonium ion,
• a ranges from 0 to 15,
• b ranges from 0 to 10, and
• the sum of a + b ranges from 1 to 25; and
- n ranges from 1 to 10.

13. Cosmetic composition according to any of claims 1 to 12, comprising at least one hydrophilic gelling agent chosen from the group consisting of cross-linked and/or neutralized polyacrylamides and polymers and copolymers of 2-acrylamido 2-methylpropane sulfonic acid, polysaccharides and mixtures thereof.

14. Cosmetic composition according to claim 13, wherein the hydrophilic gelling agent is chosen from the group consisting of cross-linked ammonium acrylamido-2-methylpropane sulfonate polymers, cross-linked sodium acrylamide/acrylamido-2-methylpropane sulfonate copolymers, and mixtures thereof.

15. Cosmetic composition according to claim 13, wherein the hydrophilic gelling agent is xanthan gum.

16. Cosmetic composition according to any of claims 1 to 15, comprising at least one filler.

17. Cosmetic composition according to any of claims 1 to 16, in the form of a foundation.

18. Non-therapeutic makeup and/or skincare method comprising a step for applying at least one layer of a cosmetic composition according to any of claims 1 to 17 onto the skin.

## Patentansprüche

1. Kosmetische Zusammensetzung, die ein physiologisch verträgliches Medium umfasst, in der Form einer Öl-in-Wasser-Emulsion, welche enthält:
- mindestens ein assoziatives Polyurethan, das mit der folgenden Formel (I) übereinstimmt: 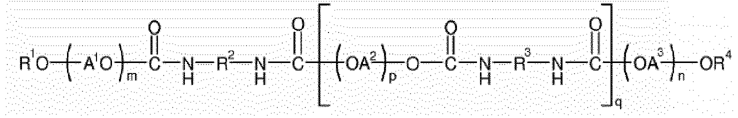 in der:
* R¹ und R⁴ unabhängig voneinander für einen geradkettigen Kohlenwasserstoffrest stehen, der 1 bis 30 Kohlenstoffatome umfasst,
* A¹, A² und A³ unabhängig voneinander für einen geradkettigen oder verzweigten Alkylenrest stehen, der 2 bis 4 Kohlenstoffatome aufweist;
* m und n unabhängig voneinander für eine ganze Zahl zwischen 35 und 500 stehen,
* p für eine ganze Zahl zwischen 5 und 500 steht,
* q für eine ganze Zahl zwischen 1 und 8 steht,
* R² und R³ unabhängig voneinander für einen geradkettigen oder verzweigten zweiwertigen Kohlenwasserstoffrest stehen, der 1 bis 30 Kohlenstoffatome umfasst,
- und Pigmentteilchen, die mit mindestens einer lipophilen Verbindung beschichtet sind, wobei die durchschnittliche Größe der Teilchen mehr als 100 nm beträgt,
und wobei sich die Pigmentteilchen in der Ölphase der Emulsion befinden, und wobei die beschichteten Pigmentteilchen Eisenoxid- und/oder Titanoxidteilchen sind, die mit einer lipophilen Verbindung beschichtet sind, ausgewählt aus der Gruppe bestehend aus Dinatriumstearoylglutamat, Isopropyltrisostearyltitanat, Dimethicon, Triethoxycaprylylsilan, hydriertem Lecithin und Mischungen davon.

2. Kosmetische Zusammensetzung nach Anspruch 1, die in Verhältnis zum Gesamtgewicht der Zusammensetzung 0,01 Gewichts-% bis 10 Gewichts-% assoziativen Polyurethan-Wirkstoff umfasst, der die Formel (I) aufweist.

3. Kosmetische Zusammensetzung nach Anspruch 1 oder 2, wobei in Formel (I) A¹, A² und A³ für einen Ethylenrest stehen.

4. Kosmetische Zusammensetzung nach einem der Ansprüche 1 bis 3, wobei in Formel (I) R¹ und R⁴ unabhängig voneinander für eine geradkettige Alkylgruppe stehen, die 8 bis 30 Kohlenstoffatome umfasst.

5. Zusammensetzung nach Anspruch 4, wobei in Formel (I) R¹ und R⁴ für eine geradkettige Alkylgruppe stehen, die 18 Kohlenstoffatome umfasst.

6. Kosmetische Zusammensetzung nach einem der Ansprüche 1 bis 5, wobei in Formel (I) m und n unabhängig voneinander für eine ganze Zahl zwischen 50 und 200 stehen.

7. Kosmetische Zusammensetzung nach einem der Ansprüche 1 bis 6, die in Verhältnis zum Gesamtgewicht der Verbindung 0,1 Gewichts-% bis 30 Gewichts-% Pigmentteilchen umfasst, die mit mindestens einer lipophilen Verbindung beschichtet sind.

8. Kosmetische Zusammensetzung nach einem der Ansprüche 1 bis 7, die mindestens ein Tensid umfasst.

9. Kosmetische Zusammensetzung nach einem der Ansprüche 1 bis 8, die mindestens ein anionisches Tensid umfasst.

10. Kosmetische Zusammensetzung nach Anspruch 9, wobei das anionische Tensid ein Silikontensid ist, insbesondere Polydimethylsiloxan mit einer Oxyethylen-/Oxypropylen-Endung.

11. Kosmetische Zusammensetzung nach Anspruch 9, wobei das anionische Tensid ein polyoxyethylenierter Alkyl- und Polyalkylester von Glycerin, insbesondere Zitronensäure- und Stearinsäureester von Polyglycerin ist.

12. Kosmetische Zusammensetzung nach Anspruch 9, wobei das anionische Tensid ein Geminitensid ist, insbesondere der Formel (I): 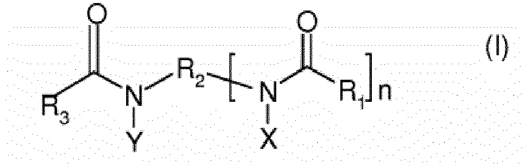 in der:
- R¹ und R³ unabhängig voneinander einen Alkylrest bezeichnen, der 1 bis 25 Kohlenstoffatome enthält;
- R² einen Spacer bezeichnet, der aus einer geradkettigen oder verzweigten Alkylenkette besteht, die 1 bis 12 Kohlenstoffatome enthält;
- X und Y unabhängig voneinander eine -(C₂H₄O)ₐ-(C₃H₆O)_{b}Z-Gruppe bezeichnen, worin:
• Z ein Wasserstoffatom oder einen -CH₂-COOM-, SO₃M-, -P(O)(OM)₂-,-C₂H₄-SO₃M-, -C₃H₆SO₃M- oder -CH₂(CHOH)₄CH₂OH-Rest bezeichnet, worin M und M' für H oder ein Alkalimetall- oder Erdalkalimetall- oder Ammonium- oder Alkanolammoniumion stehen,
• a von 0 bis 15 reicht,
• b von 0 bis 10 reicht, und
• die Summe aus a + b von 1 bis 25 reicht; und
- n von 1 bis 10 reicht.

13. Kosmetische Zusammensetzung nach einem der Ansprüche 1 bis 12, die mindestens ein hydrophiles Geliermittel umfasst, ausgewählt aus der Gruppe bestehend aus quervernetzten und/oder neutralisierten Polyacrylamiden und Polymeren und Copolymeren von 2-Acrylamido-2-methylpropansulfonsäure, Polysacchariden und Mischungen davon.

14. Kosmetische Zusammensetzung nach Anspruch 13, wobei das hydrophile Geliermittel ausgewählt ist aus der Gruppe bestehend aus quervernetzten Ammoniumacrylamido-2-methylpropansulfonat-Polymeren, quervernetzten Natriumacrylamid-/Acrylamido-2-methylpropansulfonat-Copolymeren, und Mischungen davon.

15. Kosmetische Zusammensetzung nach Anspruch 13, wobei das hydrophile Geliermittel Xanthangummi ist.

16. Kosmetische Zusammensetzung nach einem der Ansprüche 1 bis 15, die mindestens einen Füllstoff umfasst.

17. Kosmetische Zusammensetzung nach einem der Ansprüche 1 bis 16 in der Form einer Foundation.

18. Nicht therapeutisches Schmink- und/oder Hautpflegeverfahren, das einen Schritt zum Auftragen von mindestens einer Schicht einer kosmetischen Zusammensetzung nach einem der Ansprüche 1 bis 17 auf die Haut umfasst.

## Revendications

1. Composition cosmétique comprenant un milieu physiologiquement acceptable, sous la forme d'une émulsion huile-dans-eau, contenant :
- au moins un polyuréthane associatif répondant à la formule (I) suivante : 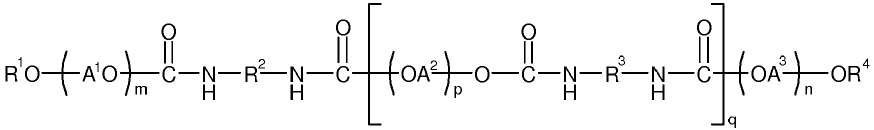 dans laquelle :
* R¹ et R⁴ représentent, indépendamment l'un de l'autre, un radical hydrocarboné linéaire comprenant de 1 à 30 atomes de carbone,
* A¹, A² et A³ représentent, indépendamment l'un de l'autre, un radical alkylène, linéaire ou ramifié, ayant de 2 à 4 atomes de carbone ;
* m et n représentent, indépendamment l'un de l'autre, un nombre entier compris entre 35 et 500,
* p représente un nombre entier compris entre 5 et 500,
* q représente un nombre compris entre 1 et 8,
* R² et R³ représentent, indépendamment l'un de l'autre, un radical hydrocarboné bivalent, linéaire ou ramifié, comprenant de 1 à 30 atomes de carbone,
- et des particules de pigment enrobé par au moins un composé lipophile, la taille moyenne desdites particules étant supérieure à 100 nm et lesdites particules étant présentes dans la phase huileuse de ladite émulsion,
dans laquelle les particules de pigment enrobé sont des particules d'oxydes de fer et/ou d'oxyde de titane enrobées par un composé lipophile choisi dans le groupe constitué du disodium stéaroyl glutamate, du trisostéaryle titanate d'isopropyle, de la diméthicone, du triéthoxy caprylylsilane, de la lécithine hydrogénée et de leurs mélanges.

2. Composition cosmétique selon la revendication 1, comprenant de 0,01% à 10% en poids de matière active de polyuréthane associatif de formule (I) par rapport au poids total de ladite composition.

3. Composition cosmétique selon la revendication 1 ou 2, dans laquelle, dans la formule (I), A¹, A² et A³ représentent un radical éthylène.

4. Composition cosmétique selon l'une quelconque des revendications 1 à 3, dans laquelle, dans la formule (I), R¹ et R⁴ représentent, indépendamment l'un de l'autre, un groupe alkyle linéaire comprenant de 8 à 30 atomes de carbone.

5. Composition selon la revendication 4, dans laquelle, dans la formule (I), R¹ et R⁴ représentent un groupe alkyle linéaire comprenant 18 atomes de carbone.

6. Composition cosmétique selon l'une quelconque des revendications 1 à 5, dans laquelle, dans la formule (I), m et n représentent, indépendamment l'un de l'autre, un nombre entier compris entre 50 et 200.

7. Composition cosmétique selon l'une quelconque des revendications 1 à 6, comprenant de 0,1% à 30% en poids de particules de pigment enrobé par au moins un composé lipophile par rapport au poids total de ladite composition.

8. Composition cosmétique selon l'une quelconque des revendications 1 à 7, comprenant au moins un tensioactif.

9. Composition cosmétique selon l'une quelconque des revendications 1 à 8, comprenant au moins un tensioactif non ionique.

10. Composition cosmétique selon la revendication 9, dans laquelle le tensioactif non ionique est un tensioactif siliconé, en particulier du polydiméthylsiloxane avec une terminaison oxyéthylène/oxypropylène.

11. Composition cosmétique la revendication 9, dans laquelle le tensioactif non ionique est un ester d'alkyle et polyalkyle polyoxyéthyléné de glycérol, en particulier l'ester citrique et stéarique de polyglycérol.

12. Composition cosmétique selon la revendication 9, dans laquelle le tensioactif non ionique est un tensioactif géminé, en particulier de formule (I): 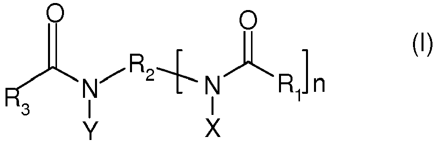 dans laquelle :
- R₁ et R₃ représentent, indépendamment l'un de l'autre, un radical alkyle contenant de 1 à 25 atomes de carbone ;
- R₂ représente un espaceur constitué d'une chaîne alkylène linéaire ou ramifiée contenant de 1 à 12 atomes de carbone ;
- X and Y représentent, indépendamment l'un de l'autre, un groupe -(C₂H₄O)ₐ-(C₃H₆O)_{b}Z, où :
• Z représente un atome d'hydrogène ou un radical -CH₂-COOM, -SO₃M, -P(O)(OM)₂, -C₂H₄-SO₃M, -C₃H₆-SO₃M ou -CH₂(CHOH)₄CH₂OH, où M et M' représentent H ou un metal alcalin ou un métal alcalino-terreux ou un ion ammonium ou alcanolammonium,
• a varie de 0 à 15,
• b varie de 0 à 10, et
• la somme a + b varie de 1 à 25 ; et
- n varie de 1 à 10.

13. Composition cosmétique selon l'une quelconque des revendications 1 à 12, comprenant au moins un gélifiant hydrophile choisi dans le groupe constitué des polyacrylamides et des polymères et copolymères d'acide 2-acrylamido 2-méthylpropane sulfonique, réticulés et/ou neutralisés, des polysaccharides et de leurs mélanges.

14. Composition cosmétique selon l'une quelconque des revendications 1 à 13, dans laquelle l'agent gélifiant hydrophile est choisi dans le groupe constitué des polymères réticulés d'acrylamido-2-méthylpropane sulfonate d'ammonium, des copolymères réticulés acrylamide/acrylamido-2-méthylpropane sulfonate de sodium et de leurs mélanges.

15. Composition cosmétique selon l'une quelconque des revendications 1 à 13, dans laquelle l'agent gélifiant hydrophile est la gomme de xanthane.

16. Composition cosmétique selon l'une quelconque des revendications 1 à 15, comprenant au moins une charge.

17. Composition cosmétique selon l'une quelconque des revendications 1 à 16, sous la forme d'un fond de teint.

18. Procédé non thérapeutique de maquillage et/ou de soin de la peau comprenant une étape d'application sur la peau d'au moins une couche d'une composition cosmétique selon l'une quelconque des revendications 1 à 17.
